# EUROPEAN PATENT APPLICATION

(11) **EP 4 302 771 A1**
(43) Date of publication of application: **10.01.2024**
(21) Application number: 22763401.1
(22) Date of filing: 03.03.2022
(51) Int. Cl.: A61K 35/33, A61K 35/34, A61K 35/42, A61P 3/00, A61P 7/10, A61P 11/00, A61P 37/04, A61P 43/00, C12N 5/071, C12N 5/077, C12N 15/12

(54) **LYMPHANGIOGENESIS PROMOTING FACTOR-EXPRESSING FIBROBLAST, AND PHARMACEUTICAL COMPOSITION CONTAINING SAME**

(30) Priority: 04.03.2021 JP 2021034016; 04.03.2021 JP 2021034018
(71) Applicant: Metcela Inc., Tsuruoka-shi, Yamagata, 997-0002 (JP)
(72) Inventor: IWAMIYA, Takahiro, Tsuruoka-shi, Yamagata 997-0002 (JP)
(74) Representative: Jones, Nicholas Andrew
(86) International application number: PCT/JP2022/009205
(87) International publication number: WO 2022/186349

(57) **Abstract**

A fibroblast highly expressing ADM and/or HHEX has been found as a cell having lymphangiogenesis ability, and a procedure for treating fibrosis, a procedure for ameliorating a condition of lymphangiogenesis ability reduced, and procedures for modulating homeostasis of a tissue fluid, transport of lipid and/or vitamin, and immunological surveillance, and for treating organ failure, by use of a pharmaceutical composition including the fibroblast, have been found. In addition, it has been found that an adult-derived fibroblast highly expressing ADM and increasing expression of VCAM-1 is obtained by culturing a human fibroblast with a medium to which TNF-α and IL-4 are added and it has been found that a fibroblast highly expressing ADM is provided, and a procedure for treating a pulmonary disease by use of the fibroblast has been found.

## Description

### TECHNICAL FIELD

The present invention relates to a lymphangiogenesis promoting factor-expressing fibroblast, and in particular, relates to a lymphangiogenesis promoting factor-expressing fibroblast expressing specified gene and/or protein (adrenomedullin (hereinafter, also referred to as "ADM") and/or Hematopoietically-expressed homeobox protein (hereinafter, also referred to as "HHEX")), and a pharmaceutical composition including the same.

### BACKGROUND ART

Lymphatic vessels are duct networks that run along the vascular system and that are distributed throughout the body. Lymphatic vessels play important roles in treatment/amelioration of fibrosis (Non-Patent Document 1), the maintenance of homeostasis of living tissue fluids (Non-Patent Document 2), lipid and vitamin transport (Non-Patent Document 3), and the modulation of immunological surveillance (Non-Patent Document 4). Lymphatic vessels also allow for the maintenance of body fluid balance in the heart in order to maintain normal cardiac output.

In recent years, the formation-promoting effect of lymphatic vessels (hereinafter, referred to as "lymphangiogenesis") has been reported to be useful in a therapeutic target for cardiac output restoration after myocardial ischemia and myocardial infarction, and has also attracted attention as a therapeutic target for reduction of edema (Non-Patent Document 1).

ADM is a cardioprotective peptide serving important functions in the development of the cardiovascular system and the lymphatic system. ADM has been reported to stabilize lymphatic endothelial cell barrier and promote lymphangiogenesis (Non-Patent Document 5). ADM has also been reported to be intravenously administered in clinical use to an acute myocardial infarction patient, thereby resulting in significant amelioration of the cardiovascular system (Non-Patent Document 6).

HHEX has been not only known as a direct transcription factor for VEGFA, VEGFR1 and VEGFR2, but also reported to regulate lymphangiogenesis via the VEGFC/FLT4/PROX1 signal (Non-Patent Document 7).

Interstitial pneumonia refers to any pathological condition where virus infections such as COVID-19 infection (COVID-19), risk factors such as aging or smoking, diseases such as collagenosis or sarcoidosis, and/or the like cause overdrive (cytokine storm) of inflammatory responses of alveolus to thereby lead to progress of fibrosis in alveolus and thus the loss of gas exchange function in the lung (Non-Patent Document 8). In interstitial pneumonia, any symptom whose onset factor cannot be identified is called idiopathic interstitial pneumonia (IIP), and such a disease is specified as "intractable disease (specified disease)". Idiopathic pulmonary fibrosis (IPF) with which 80 to 90% of IIP patients are concerned exhibits resistance particularly to current therapies, and is known as a poor-prognosis disease as compared with many cancers because the average survival period after diagnosis is three to five years and the average survival period after acute exacerbation is within two months. Developments of therapeutic methods of interstitial pneumonia and pulmonary fibrosis including IPF are advanced in view of such circumstances, and some pharmaceutical products are clinically applied. For example, Pirfenidone has been demonstrated to potentially delay decreases in forced ventilation capacity (FVC) and 6-min walk distance, and decrease the mortality of specified patients. Nintedanib has also been demonstrated to likely delay a decrease in FVC and decrease mortality. However, such therapeutic methods allow for no observation of any amelioration of respiratory symptoms, and lead to no remarkable improvement in acute exacerbation rate. Accordingly, it has been revealed that the lung function is ongoingly ever-worsening.

In order to overcome these problems, several tens of novel therapeutic products including connective tissue growth factors, interleukin (IL)-4 and IL-13 antibodies, galectin-3 inhibitors, lysophosphatidic acid receptor antagonists, phosphoinositide 3 kinase pathway inhibitors, and mesenchymal stem cells (MSC) have been developed and clinical trials of some thereof have progressed, but there is currently no effective therapeutic method of acute exacerbation of pulmonary fibrosis.

It has been recently reported that a pathophysiological onset factor of pulmonary fibrosis is activation of chronic and repetitive inflammatory response, as in asthma and chronic obstructive pulmonary disease (COPD). While the healthy lung allows inflammatory cytokine to take a role as control of lung inflammatory response to virus infection or injury and thus contribute to tissue repair and maintenance of homeostasis, it has been indicated that inflammatory cytokine is excessively produced in the case of pulmonary fibrosis to lead to overdrive (cytokine storm) of inflammatory response and thus induction of formation of muscle fibroblast foci, resulting in excess deposition of extracellular matrices (ECM) and then induction of fibrosis (Non-Patent Document 9). Such induction of fibrosis due to excess inflammatory response is suggested to be associated with structural/functional changes of lymphatic vessels (Non-Patent Document 10).

Lymphatic vessels are configured from single lymphatic endothelial cell (LEC) walls having non-continuous basal membranes, and function as the transport pathway of antigens and antigen-presenting cells from peripheral tissues to lymphatic nodes, or as the clearance of interstitial fluids. Lymphatic vessel disorders are caused in fibrosis, and, for example, it has been reported that a remarkable decrease in lymphatic density correlates to induction of fibrosis in a radiation-induced IPF animal model (Non-Patent Document 11) and it has been reported that platelet-derived growth factor receptor (PDGFR)-β-positive wall cells inhibit discharge of hyaluronan being an important molecule in injury and repair of the lung to thereby induce a lymphatic vessel disorder in the lung in a bleomycin-induced IPF animal model (Non-Patent Document 12). On the other hand, human IPF leads to an increase in lymphatic density in a lung tissue, but lymphatic vessels formed have structural/functional defects, and localization of PDGFR-positive wall cells and formation of lymphatic vessels intermittently destroyed are observed (Non-Patent Document 13). It has also been reported that overexpression of transforming growth factor (TGF)-β1 by fibrosis-induced fibroblasts in wound healing inhibits proliferation and function of lymphatic endothelial cells and selectively destroys lymphatic vessels (Non-Patent Document 14).

The present inventors heretofore have found a heart-derived vascular cell adhesion molecule-1 (VCAM-1, CD106)-positive fibroblast that can efficiently induce cardiac lymphangiogenesis and significantly improve the force of cardiac contraction in heart failure (Non-Patent Document 15), and also have found a procedure for producing an adult heart-derived VCAM-1-positive fibroblast (Patent Document 1).

### RELATED ART DOCUMENTS

### PATENT DOCUMENT

Patent Document 1: International Publication No. WO 2020/045547

### NON PATENT DOCUMENTS

Non-Patent Document 1: Circulation. 2016; 133: 1484-1497
Non-Patent Document 2: Bull N Y Acad Med., 1938; 14: 231-251
Non-Patent Document 3: Am J Physiol Endocrinol Metab., 2009; 296: E1183-E1194
Non-Patent Document 4: Front Immunol., 2016; 7: 613
Non-Patent Document 5: Peptides, 2008; 29: 2243-2249
Non-Patent Document 6: J Cardiovasc Pharmacol., 2010; 56: 413-419
Non-Patent Document 7: Nat Commun., 2018; 9; Article number 2704
Non-Patent Document 8: Ann Intern Med., 2020; 172(9): 629-632
Non-Patent Document 9: Semin Respir Crit Care Med., 2006; 27: 600-612
Non-Patent Document 10: Lymphat Res Biol., 2009; 7: 197-203
Non-Patent Document 11: Lymphat Res Biol., 2014; 12: 238-250
Non-Patent Document 12: Blood, 2012; 119: 5931-5942
Non-Patent Document 13: Lymphat Res Biol., 2010; 8: 199-207
Non-Patent Document 14: Am J Physiol - Heart Circ Physiol., 2008; 295: 2113-2127
Non-Patent Document 15: PLoS One., 2020; 15: e0237810
Non-Patent Document 16: Pulm Pharmacol Ther., 2012 Aug; 25: 281-285
Non-Patent Document 17: Stem Cells Transl Med. 2017; 6(10): 1905-1916

### SUMMARY OF THE INVENTION

### PROBLEMS TO BE SOLVED BY THE INVENTION

An object of the present invention is to provide a fibroblast having lymphangiogenesis ability and a pharmaceutical composition including the fibroblast.

### MEANS FOR SOLVING THE PROBLEMS

The present inventors have made studies in order to solve the above problems, have found that a fibroblast contacted with TNF-α and IL-4 significantly enhances the gene expression level(s) of ADM and/or HHEX, and have completed the present invention.

In other words, the present invention includes the following first aspect (Invention A).
(A1) A pharmaceutical composition comprising a fibroblast highly expressing ADM and/or HHEX.
(A2) The pharmaceutical composition according to (A1), wherein the fibroblast is an adult-derived fibroblast.
(A3) The pharmaceutical composition according to (A1) or (A2), wherein gene expression level(s) of ADM and/or HHEX in the fibroblast are/is 1.20 times or higher as compared with a control fibroblast.
(A4) The pharmaceutical composition according to any of (A1) to (A3), for treating fibrosis.
(A5) The pharmaceutical composition according to any of (A1) to (A4), for amelioration of a condition of lymphangiogenesis ability reduced.
(A6) The pharmaceutical composition according to any of (A1) to (A5), for amelioration of a disorder of homeostasis in a tissue fluid.
(A7) The pharmaceutical composition according to any of (A1) to (A6), for reduction of edema.
(A8) The pharmaceutical composition according to any of (A1) to (A7), for amelioration of a disorder of transportability of lipid and/or vitamin.
(A9) The pharmaceutical composition according to any of (A1) to (A8), for activation of an immunological surveillance mechanism.
(A10) The pharmaceutical composition according to any of (A1) to (A9), wherein the pharmaceutical composition is an injectable composition.
(A11) The pharmaceutical composition according to any of (A1) to (A9), wherein the fibroblast is constructed as a planar or three-dimensional cellular tissue.
(A12) The pharmaceutical composition according to any of (A1) to (A11), wherein the fibroblast is a cardiac fibroblast.

The present invention includes the following second aspect (Invention B).
(B1) A method for treating fibrosis in a subject, the method comprising administrating or transplanting a pharmaceutical composition comprising a fibroblast highly expressing ADM and/or HHEX, to the subject.
(B2) A method for ameliorating a condition of lymphangiogenesis ability reduced, in a subject, the method comprising administrating or transplanting a pharmaceutical composition comprising a fibroblast highly expressing ADM and/or HHEX, to the subj ect.
(B3) A method for ameliorating a disorder of homeostasis in a tissue fluid, in a subject, the method comprising administrating or transplanting a pharmaceutical composition comprising a fibroblast highly expressing ADM and/or HHEX, to the subject.
(B4) A method for reducing edema in a subject, the method comprising administrating or transplanting a pharmaceutical composition comprising a fibroblast highly expressing ADM and/or HHEX, to the subject.
(B5) A method for ameliorating a disorder of transportability of lipid and/or vitamin, in a subject, the method comprising administrating or transplanting a pharmaceutical composition comprising a fibroblast highly expressing ADM and/or HHEX, to the subj ect.
(B6) A method for activating an immunological surveillance mechanism in a subject, the method comprising administrating or transplanting a pharmaceutical composition comprising a fibroblast highly expressing ADM and/or HHEX, to the subject.
(B7) A method for treating organ failure in a subject, the method comprising administrating or transplanting a pharmaceutical composition comprising a fibroblast highly expressing ADM and/or HHEX, to the subject.

The present invention includes the following third aspect (Invention C).
(C1) Use of a pharmaceutical composition comprising a fibroblast highly expressing ADM and/or HHEX, as an injection for administration to a failed organ tissue and/or its peripheral region, or a fibrotic tissue and/or its peripheral region.
(C2) Use of a pharmaceutical composition comprising a fibroblast highly expressing ADM and/or HHEX, as a transplantation material for transplantation to a failed organ tissue and/or its peripheral region, or a fibrotic tissue and/or its peripheral region.

The present invention includes the following fourth aspect (Invention D).
(D1) A method for producing a fibroblast highly expressing ADM, the method comprising:
   contacting TNF-α and IL-4 with a fibroblast; and
   culturing the fibroblast contacted, under a condition where ADM is capable of being highly expressed.
(D2) The method according to (D1), wherein the fibroblast highly expresses VEGF-C.
(D3) The method according to (D1) or (D2), wherein the fibroblast is CD106-positive.
(D4) The method according to (D3), comprising selecting or concentrating a fibroblast highly expressing ADM, by use of an anti-CD106 antibody.
(D5) The method according to any of (D1) to (D4), wherein the fibroblast is a pulmonary fibroblast.
(D6) A fibroblast highly expressing ADM, obtained by the method according to any of (D1) to (D5).
(D7) A cell population of fibroblast comprising the fibroblast according to (D6).

The present invention includes the following fifth aspect (Invention E).
(E1) A fibroblast highly expressing ADM.
(E2) The fibroblast according to (E1), wherein the fibroblast highly expresses VEGF-C.
(E3) The fibroblast according to (E1) or (E2), wherein the fibroblast is CD106-positive.
(E4) The fibroblast according to any of (E1) to (E3), wherein gene expression level(s) of VEGF-C and/or ADM in the fibroblast are/is 1.20 times or higher as compared with a control fibroblast.
(E5) The fibroblast according to any of (E1) to (E4), wherein the fibroblast is a pulmonary fibroblast.
(E6) A cell population of fibroblast comprising the fibroblast according to any of (E1) to (E5).
(E7) A cell population comprising a CD106-positive pulmonary fibroblast, wherein
the proportion (based on the number of cells) of the CD106-positive pulmonary fibroblast in the total fibroblast contained in the cell population is more than 18.01%.

The present invention includes the following sixth aspect (Invention F).
(F1) A pharmaceutical composition comprising the fibroblast according to any of (D6) and (E1) to (E5) or the cell population according to any of (D7) and (E6) to (E7).
(F2) The pharmaceutical composition according to any of (F1), for treating a pulmonary disease.
(F3) The pharmaceutical composition according to (F2), wherein the pulmonary disease is pulmonary fibrosis or interstitial pneumonia.
(F4) The pharmaceutical composition according to (F1), for amelioration of a condition of lymphangiogenesis ability reduced.
(F5) The pharmaceutical composition according to any of (F1) to (F4), wherein the pharmaceutical composition is an injectable composition.
(F6) The pharmaceutical composition according to any of (F1) to (F4), wherein the fibroblast or the cell population is constructed as a planar or three-dimensional cellular tissue.

The present invention includes the following seventh aspect (Invention G).
(G1) A method for treating a pulmonary disease in a subject, the method comprising administrating or transplanting the fibroblast according to any of (D6) and (E1) to (E5), the cell population according to any of (D7) and (E6) to (E7) or the pharmaceutical composition according to (F1), to the subject.
(G2) The method according to (G1), wherein the pulmonary disease is pulmonary fibrosis or interstitial pneumonia.
(G3) A method for ameliorating a condition of lymphangiogenesis ability reduced, in a subject, the method comprising administrating or transplanting the fibroblast according to any of (D6) and (E1) to (E5), the cell population according to any of (D7) and (E6) to (E7) or the pharmaceutical composition according to (F1), to the subject.

The present invention includes the following eighth aspect (Invention H).
(H1) Use of the fibroblast according to any of (D6) and (E1) to (E5), the cell population according to any of (D7) and (E6) to (E7), or the pharmaceutical composition according to (F1), as an injection for administration to a lung tissue and/or its peripheral region.
(H2) Use of the fibroblast according to any of (D6) and (E1) to (E5), the cell population according to any of (D7) and (E6) to (E7) or the pharmaceutical composition according to (F 1), as a transplantation material for transplantation to a lung tissue and/or its peripheral region.

### EFFECT OF THE INVENTION

The present invention can provide a pharmaceutical composition including a fibroblast having lymphangiogenesis ability, namely, a pharmaceutical composition including a cell highly expressing ADM and/or HHEX, and also can provide a procedure for treating fibrosis, a procedure for ameliorating a condition of lymphangiogenesis ability reduced, and procedures for modulating homeostasis of a tissue fluid, transport of lipid and/or vitamin, and immunological surveillance, and for treating organ failure, by use of the pharmaceutical composition. The present invention can also provide a fibroblast highly expressing ADM, and a procedure for treating a pulmonary disease by use of the cell.

### BRIEF DESCRIPTION OF THE DRAWINGS

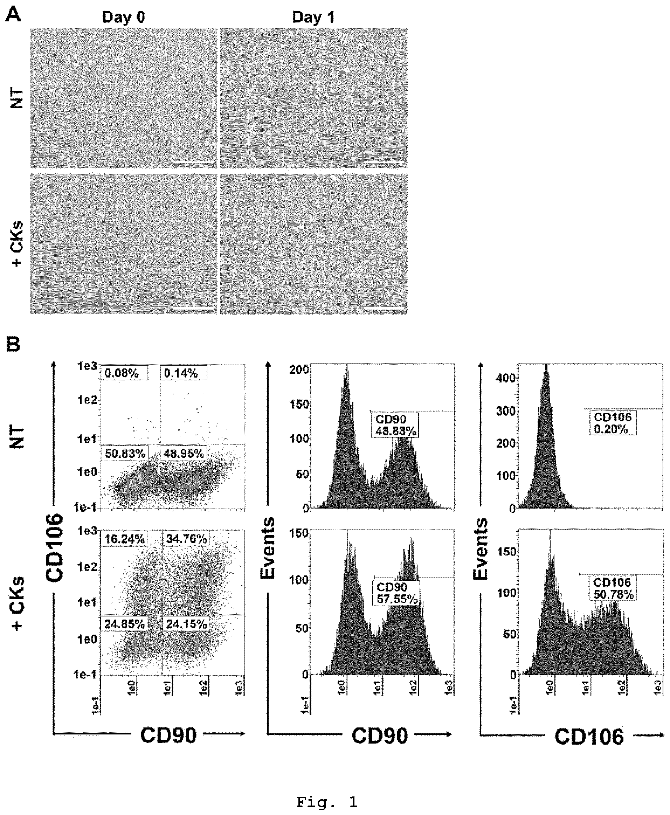

FIG. 1 illustrates characteristics of adult-derived cardiac fibroblasts under respective culturing conditions. FIG. 1A illustrates microscope photographs (scale bar: 500 µm) of cardiac fibroblasts cultured. No treatment (NT) means non-treated cardiac fibroblasts to which no cytokine is added, and +CKs means cardiac fibroblasts to which TNF-α and IL-4 are added. FIG. 1B illustrates analysis diagrams of flow cytometry. In each scatter diagram, a horizontal axis represents a CD90 expression level and a longitudinal axis represents a CD106 expression level. Histograms respectively represent a CD90 expression level and a CD106 expression level.

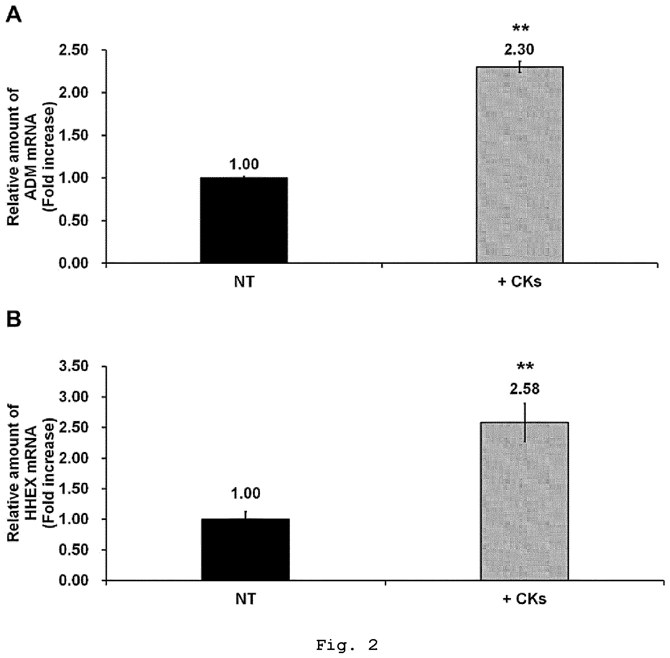

FIG. 2 illustrates the expression levels of lymphangiogenesis genes in adult-derived cardiac fibroblasts under respective culturing conditions. FIG. 2A illustrates the analysis results of real time quantitative RT-PCR (RT-qPCR) representing the mRNA expression level of ADM. The gene expression levels of ADM in fibroblasts in all groups are normalized with the gene expression levels of β-Actin, and the difference in gene expression level under each condition is represented by Fold increase. NT means non-treated cardiac fibroblasts and +CKs means cardiac fibroblasts to which TNF-α and IL-4 are added. FIG. 2B illustrates the analysis results of RT-qPCR representing the mRNA expression level of HHEX. The HHEX gene expression levels in all groups are normalized with the gene expression levels of β-Actin and are each represented by Fold increase as in ADM (**P < 0.01 vs. NT).

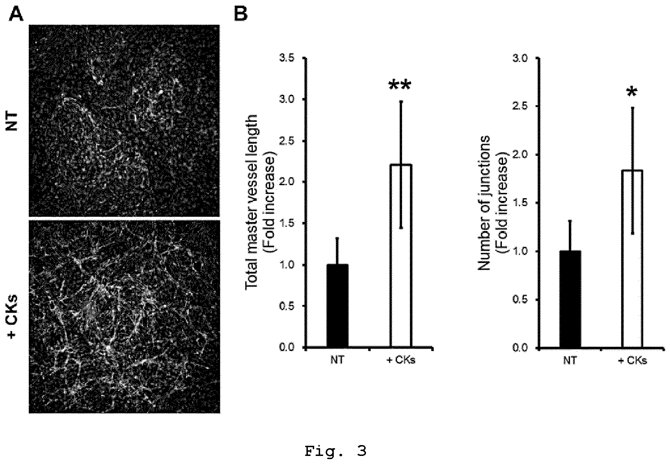

FIG. 3 illustrates the lymphangiogenesis effects of human adult-derived cardiac fibroblasts. FIG. 3A illustrates photographs indicating the vessel formation effects of cardiac lymphatic vessels with various human cardiac fibroblasts, by fluorescence microscope observation. Capillary-like structures represent Vascular Endothelial (VE)-Cadherin-positive cells, and refer to vessels formed from lymphatic endothelial cells collected from heart-derived cells. Other cell regions correspond to Vimentin and represent various cardiac fibroblasts. The nucleus is shown by Hoechst 33258 (magnification 10x). NT means non-treated cardiac fibroblasts and +CKs means cardiac fibroblasts to which TNF-α and IL-4 are added. FIG. 3B illustrates quantitative evaluations of fluorescence microscope observation images. The value of NT is assumed to be 1, and is represented by Fold increase (**P < 0.01 vs. NT, *P < 0.05 vs. NT, N = 12).

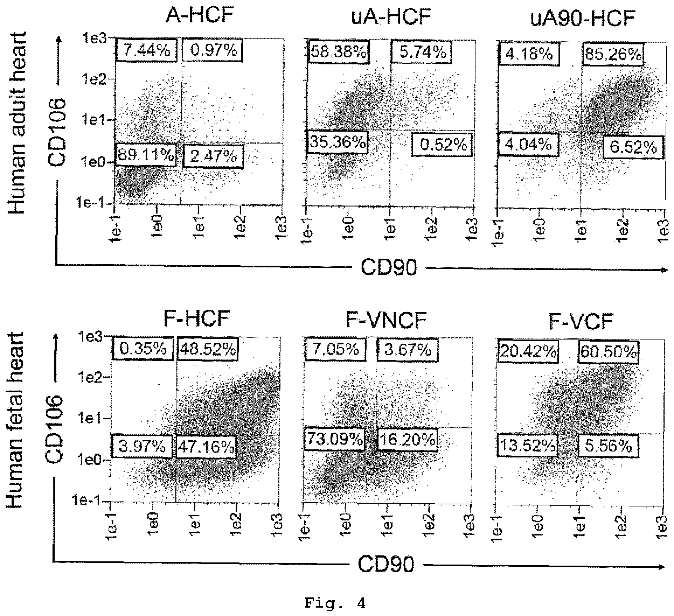

FIG. 4 illustrates analysis diagrams of flow cytometry of various fibroblasts. The upper drawings illustrate the CD90 expression levels and the CD106 expression levels in human adult-derived cardiac fibroblasts (A-HCF), adult-derived cardiac fibroblasts obtained by culturing adult-derived cardiac fibroblasts to which TNF-α and IL-4 to are added, for 72 hours (uA-HCF), and CD90-positive adult-derived cardiac fibroblasts obtained by culturing adult-derived cardiac fibroblasts to which TNF-α and IL-4 to are added, for 72 hours, and then cell sorting the resultant with CD90 as an index (uA90-HCF). The lower drawings illustrate the CD90 expression levels and the CD106 expression levels in human fetal-derived cardiac fibroblasts (F-HCF), CD106-negative fetal-derived cardiac fibroblasts (F-VNCF) and CD106-positive fetal-derived cardiac fibroblasts (F-VCF).

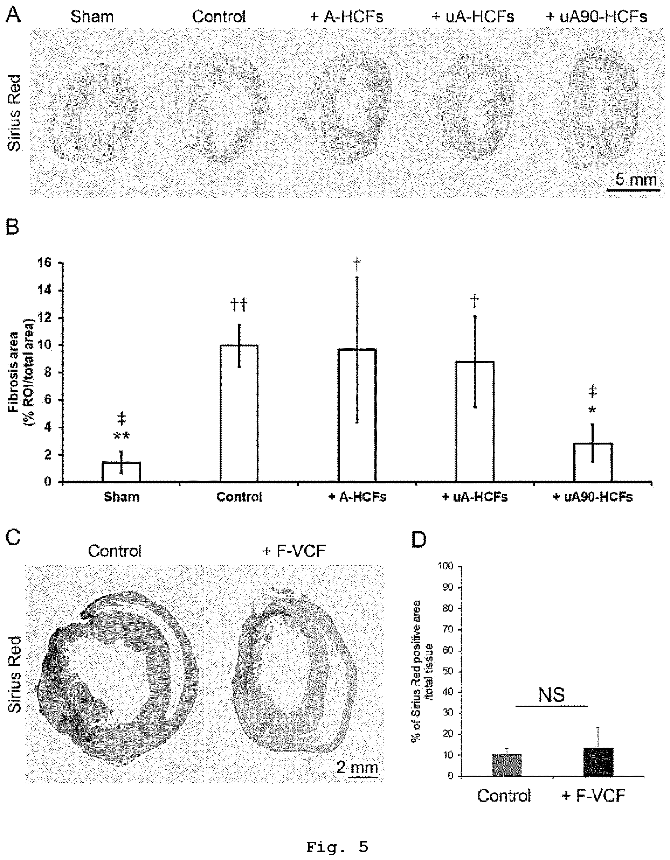

FIG. 5 illustrates the therapeutic effect of fibrosis by human adult-derived cardiac fibroblasts. FIG. 5A illustrates microscope photographs representing Sirius Red (SR) staining images of cardiac tissues of rats with chronic heart failure, to which various adult-derived cardiac fibroblasts (+A-HCFs, +uA-HCFs, +uA90-HCFs) are administered. FIG. 5B illustrates quantitative evaluations of fibrotic regions of cardiac tissues of rats with chronic heart failure, to which various adult-derived cardiac fibroblasts are administered (N = 4). FIG. 5C illustrates microscope photographs representing Sirius Red staining images of cardiac tissues of rats with chronic heart failure, to which fetal cardiac fibroblasts (+F-VCF) are administered. FIG. 5D illustrates quantitative evaluation of fibrotic regions of cardiac tissues of rats with chronic heart failure, to which fetal cardiac fibroblasts (+F-VCF) are administered (NS = not significant, N = 4, **P < 0.01 vs. Control, *P < 0.05 vs. Control, ††P < 0.01 vs. Sham, †P < 0.05 vs. Sham, ‡P < 0.05 vs. +A-HCFs).

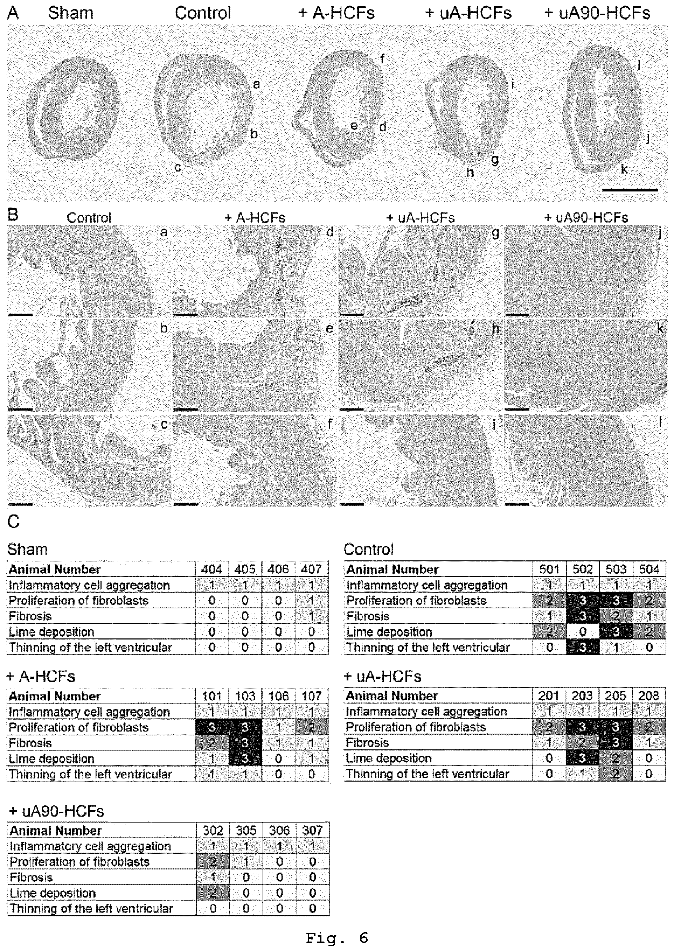

FIG. 6 illustrates the therapeutic effect of fibrosis by human adult-derived cardiac fibroblasts. FIG. 6A illustrates microscope photographs representing hematoxylin-eosin (HE) staining images of cardiac tissues of rats with chronic heart failure, to which various adult-derived cardiac fibroblasts (+A-HCFs, +uA-HCFs, +uA90-HCFs) are administered. FIG. 6B illustrates microscope photographs by enlargement of a to l sections illustrated in FIG. 6A. FIG. 6C illustrates tables representing pathological qualitative scoring. The degree of pathological change is qualitatively evaluated with No change (Score 0), Mild (Score 1), Moderate (Score 2), and Severe (Score 3). Scoring items are Inflammatory cell aggregation, Proliferation of fibroblasts, Fibrosis, Lime deposition, and Thinning of the left ventricular.

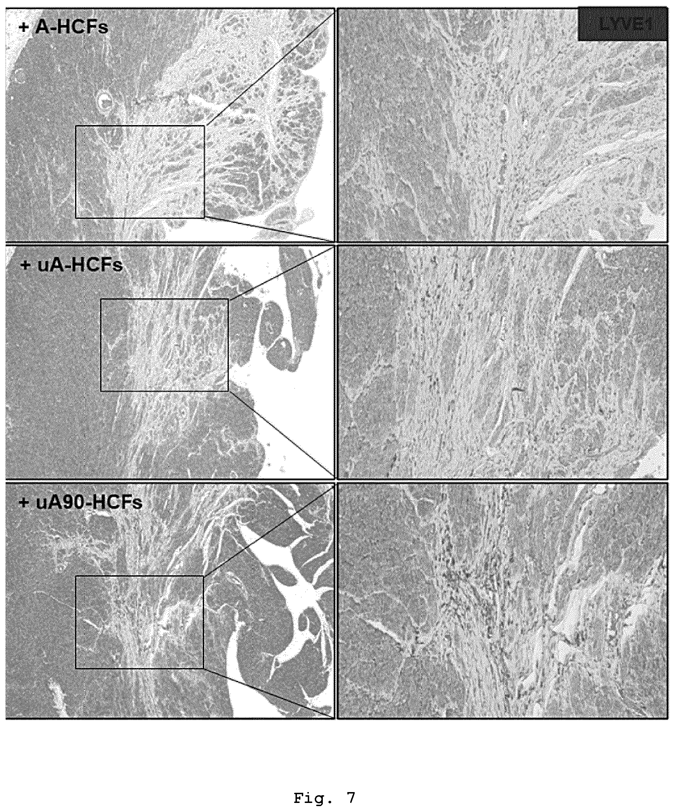

FIG. 7 illustrates the intramyocardial lymphangiogenesis effect by human adult-derived cardiac fibroblasts. The drawing illustrates microscope photographs representing Lyve-1 staining images of cardiac tissues of rats with chronic heart failure, to which adult-derived cardiac fibroblasts (+A-HCFs, +uA-HCFs, +uA90-HCFs) are administered (*in-situ* hybridization). Each panel in the right row is obtained by enlarging a rectangular frame in each panel in the left row.

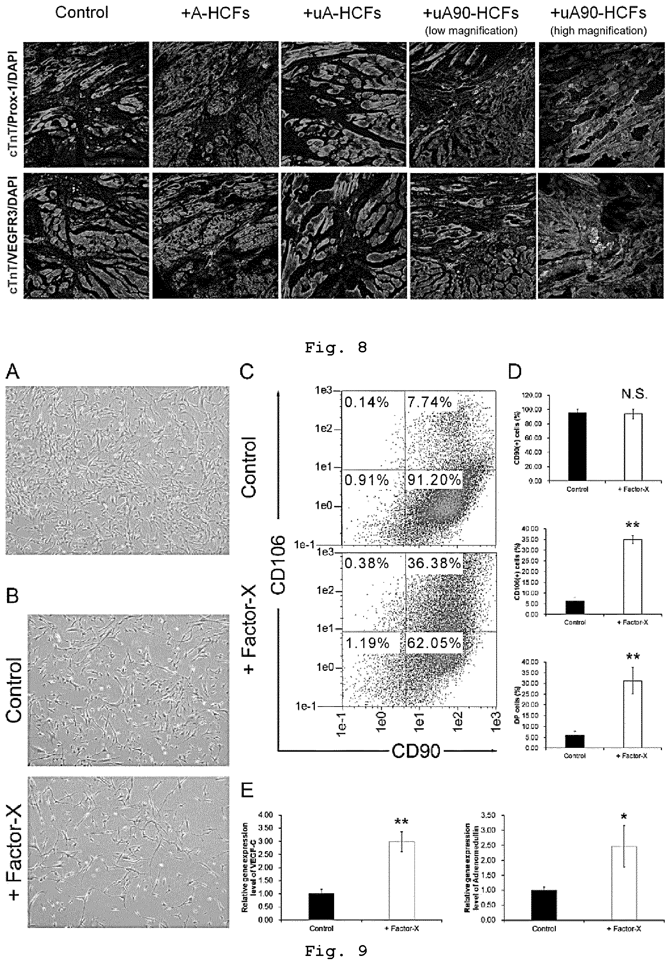

FIG. 8 illustrates localization of rat cardiac lymphatic endothelial cells. The drawing illustrates microscope photographs representing immune tissue staining images of rat cardiac tissues of chronic heart failure models to which adult-derived cardiac fibroblasts (+A-HCFs, +uA-HCFs, +uA90-HCFs) are administered. +uA90-HCFs are represented by low-magnification and high magnification photographs. The myocardial structure is shown by cTnT, the central luminal structure is shown by Prox-1 or VEGFR3, and the nucleus is shown by DAPI.
FIG. 9 illustrates the forms and lymphangiogenesis abilities of human adult-derived pulmonary fibroblasts. FIG. 9A illustrates a microscope photograph (magnification 5x) representing a bright field observation image of a human pulmonary fibroblast. FIG. 9B illustrates microscope photographs (magnification 5x) representing bright field observation images of a human pulmonary fibroblast (Control) and a human pulmonary fibroblast (+Factor-X) cultured for 24 hours with the addition of TNF-α (50 ng/mL) and IL-4 (2 ng/mL). FIGs. 9C and 9D illustrate the ratios of CD90 (fibroblast marker)- and CD106 (VCAM-1)-positive cells of various human pulmonary fibroblasts, by flow cytometry (FCM) analysis (N = 3, **P < 0.01, by Student's t-test, N.S. represents no significant difference). FIG. 9E illustrates relative comparison diagrams of gene expression levels of vascular endothelial growth factor (VEGF)-C and Adrenomedullin (ADM) in various human pulmonary fibroblasts, by RT-qPCR analysis. The drawing is represented under the assumption that the gene expression level of Control is 1 (N = 3, **P < 0.01, *P < 0.05, by Student's t-test).

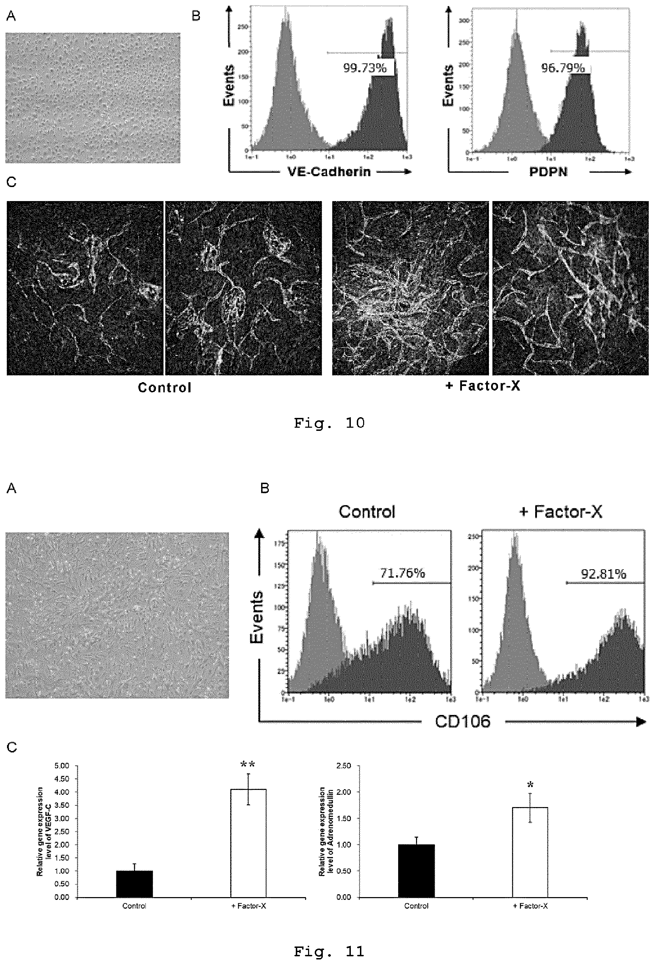

FIG. 10 illustrates the forms of lymphatic endothelial cells collected from human lung-derived cells, and the lymphangiogenesis effect of human adult-derived pulmonary fibroblasts. FIG. 10A illustrates a microscope photograph (magnification 5x) representing a bright field observation image of a human microvascular endothelial cell. FIG. 10B illustrates the ratios of Vascular Endothelial (VE)-Cadherin and Podoplanin (PDPN)-positive cells in human microvascular endothelial cells, by FCM analysis (in each graph, left peaks represent isotype control staining samples and right peaks represent various antibody staining samples). FIG. 10C illustrates photographs representing the vessel formation effect of pulmonary lymphatic vessels by various human pulmonary fibroblasts according to fluorescence microscope observation. The capillary-like structure represents VE-Cadherin-positive cells, and refers to vessels formed from lymphatic endothelial cells collected from lung-derived cells. Other cell regions mean Vimentin and represent various pulmonary fibroblasts. The nucleus is shown by Hoechst 33258 (magnification 10x).
FIG. 11 illustrates the forms and lymphangiogenesis abilities of mouse adult-derived pulmonary fibroblasts. FIG. 11A illustrates a microscope photograph (magnification 5x) representing a bright field observation image of a mouse pulmonary fibroblast. FIG. 11B illustrates the VCAM-1-positive cell ratio of mouse pulmonary fibroblasts by FCM analysis (in each graph, left peaks represent isotype control staining samples and right peaks represent various antibody staining samples). FIG. 11C illustrates relative comparison of gene expression levels of VEGF-C and ADM in various mouse pulmonary fibroblasts by RT-qPCR analysis. The drawing is represented under the assumption that the gene expression level of Control is 1 (N = 3, **P < 0.01, *P < 0.05, by Student's t-test).

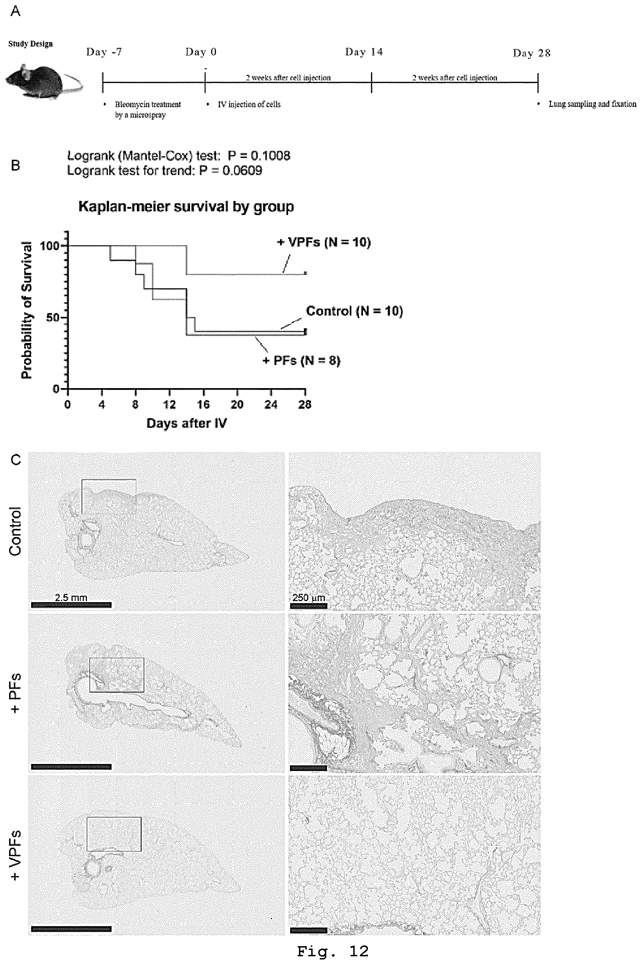

FIG. 12 illustrates the therapeutic effect of interstitial pneumonia/pulmonary fibrosis with adult-derived pulmonary fibroblasts highly expressing ADM and increasing expression of VCAM-1 (VPF). FIG. 12A illustrates an animal experimental design. Cell administration was applied to a model mouse having a pathological condition after 7 days of bleomycin solution administration, the mouse was sacrificed after 4 weeks, and the lung was collected. FIG. 12B illustrates a survival analysis diagram of each group by a Kaplan-Meier method. The dot means a break-off point. FIG. 12C illustrates microscope photographs representing of Sirius Red staining images of each group.

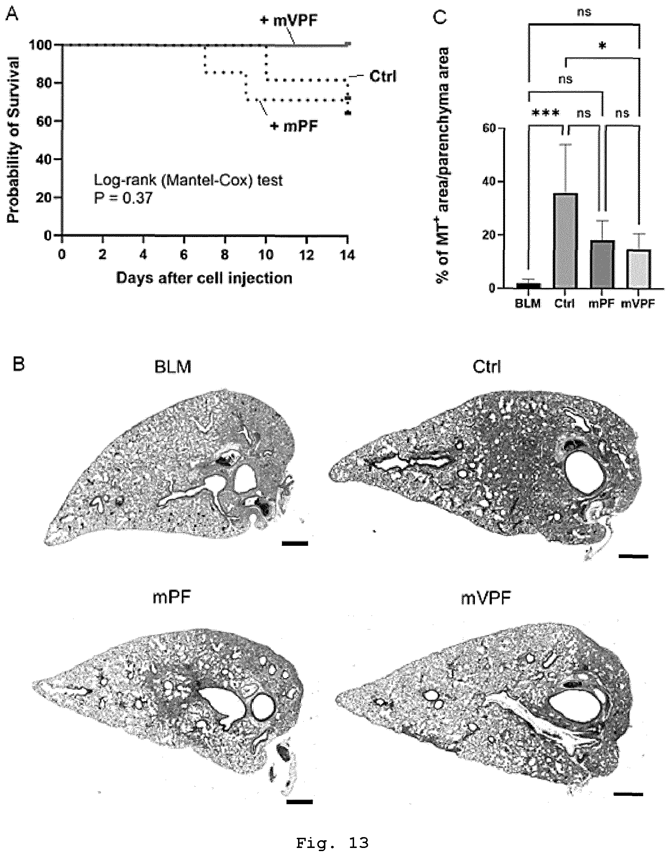

FIG. 13 illustrates the therapeutic effects by various fibroblasts, after 2 weeks of administration. FIG. 13A illustrates a survival analysis diagram. A PF administration group (+mPF) was examined at n = 7, an mVPF administration group (+mVPF) was examined at n = 5, and a physiological saline solution administration group (Ctrl) was examined at n = 11. The P value was calculated by a logrank (Mantel-Cox) test. FIG. 13B illustrates microscope photographs of a fibrotic region of the lung and an alveoli structure in each group. Lung tissues collected after 14 days of cell administration were subjected to Masson's Trichrome staining. BLM represents a lung tissue after 0 days of cell administration, and Ctrl represents a lung tissue of a physiological saline solution administration group (scale bar: 500 µm). FIG. 13C illustrates quantitative evaluation of a fibrotic region of the lung in each group (BLM: n = 5, Ctrl: n = 6, +mPF: n = 4, +mVPF: n = 5, *P < 0.05, ***P = 0.0006, ns represents no significant difference, ordinary one-way ANOVA with Tukey's multiple comparisons test).

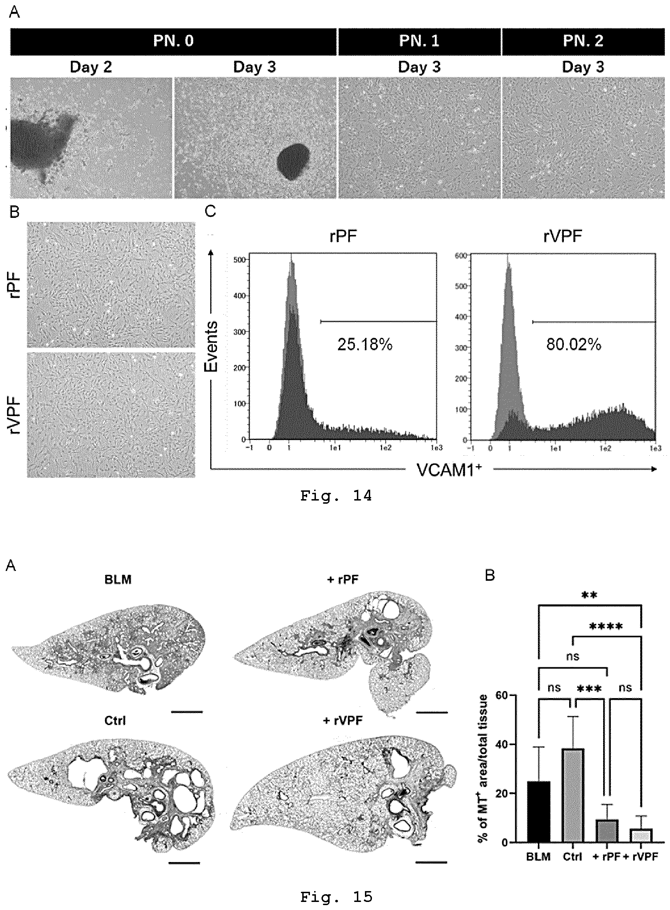

FIG. 14 illustrates cell characteristics of rPF and rVPF collected by the primary. FIG. 14A illustrates microscope photographs representing the rPF states at respective passage numbers. PN refers to the Passage number. FIG. 14B illustrates photographs representing bright field observation images of rPF and rVPF. FIG. 14C illustrates flow cytometry analysis diagrams.
FIG. 15 illustrates the therapeutic effects of various fibroblasts after 2 weeks of administration. FIG. 15A illustrates microscope photographs representing a fibrotic region of the lung and an alveoli structure of each group. Lung tissues collected after 0 days or 14 days of cell administration were subjected to Masson's Trichrome staining. BLM represents a lung tissue after 0 days of cell administration, and Ctrl represents a lung tissue of a physiological saline solution administration group. FIG. 15B illustrates a graph representing quantitative evaluation of a fibrotic region of the lung in each group (BLM: n = 5, Ctrl: n = 6, +rPF: n = 7, +rVPF: n = 8, **P < 0.01, ***P = 0.0001, ****P < 0.0001, ns represents no significant difference, calculated by ordinary one-way ANOVA with Tukey's multiple comparisons test).

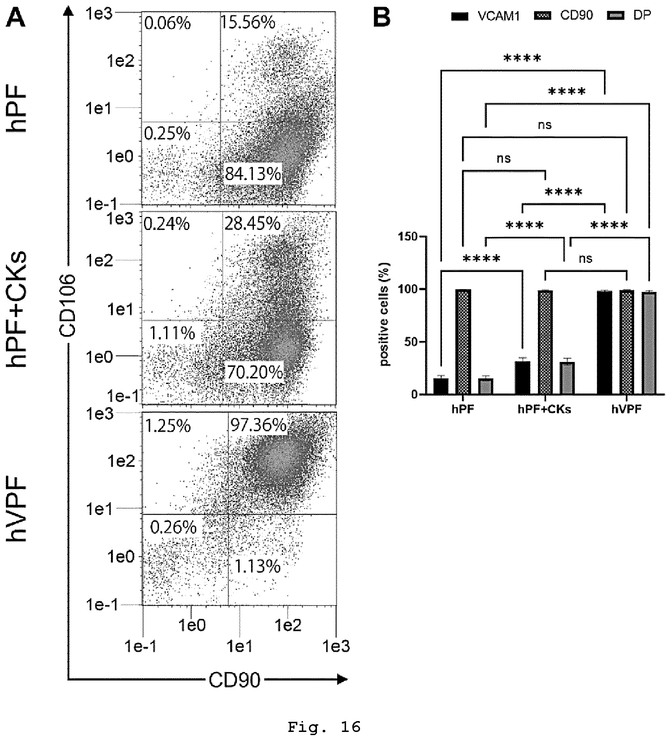

FIG. 16 illustrates CD90 (fibroblast marker)- and CD106 (VCAM-1)-positive cell ratios of various human pulmonary fibroblasts by flow cytometry (FCM) analysis (N = 5, ****P < 0.0001, ns represents no significant difference, calculated by 2-way ANOVA with Tukey's multiple comparisons test). hPF refers to human pulmonary fibroblasts, and hPF+CKs refers to human pulmonary fibroblasts cultured for 72 hours with the addition of TNF-α (50 ng/mL) and IL-4 (2 ng/mL). hVPF refers to hPF+CKs subjected to cell sorting with CD 106 as an index.

### MODE FOR CARRYING OUT THE INVENTION

Hereinafter, the present invention will be described in detail with reference to specific embodiments, but the present invention is not limited to only specific embodiments indicated.

### <Pharmaceutical composition including fibroblast highly expressing ADM and/or HIHEX>

One embodiment of the first invention relates to a pharmaceutical composition including a fibroblast highly expressing ADM and/or HHEX.

The derivation of the fibroblast is not restricted, and a pluripotent stem cell such as an embryonic stem cell (ES cell), an induced pluripotent stem cell (iPS cell) and a Muse cell, or an adult stem cell such as a mesenchymal stem cell may be differentiated and used. A primary cell collected from an animal (including human) may be used, and an established cell may also be used.

The fibroblast may be derived from an organ, such as heart, kidney, skin, or lung.

The fibroblast is preferably adult-derived without any particular limitation.

A fibroblast from which a fibroblast highly expressing ADM and/or HHEX is derived may originally express ADM and/or HHEX at certain level(s), or may not express ADM and/or HHEX at all.

The fibroblast in the present embodiment may be a cell expressing a cell surface marker expressed in a usual fibroblast, or furthermore may be a cell additionally expressing other marker or not expressing some markers by the cell production method of the present invention described below.

The "highly expressing ADM" is not particularly limited, but means that, for example, when the mRNA expression level in a control fibroblast is assumed to be 1 in the measurement of the mRNA expression level with RT-qPCR, the mRNA expression level of ADM in the fibroblast in the pharmaceutical composition of the present embodiment is preferably increased by 1.20 times or more, 1.40 times or more, 1.50 times or more, 1.60 times or more, 1.70 times or more, 1.80 times or more, 1.90 times or more, 2.00 times or more, 2.30 times or more, 3.00 times or more, 4.00 times or more, 5.00 times or more, 6.00 times or more, 7.00 times or more, 8.00 times or more, 9.00 times or more, 10.0 times or more, or more times.

The mRNA expression level in a control fibroblast may be the mRNA expression level in an untreated fibroblast or may be the average mRNA expression level in the entire fibroblast after treatment.

When the protein expression level is measured by a flow cytometry method, the proportion of an ADM-positive (hereinafter, also referred to as "ADM+") fibroblast may be 0.1% or more, may be 1% or more, may be 2% or more, may be 4% or more, may be 5% or more, may be 10% or more, may be 20% or more, may be 30% or more, may be 40% or more, may be 50% or more, may be 60% or more, may be 70% or more, may be 80% or more, may be 90% or more, may be 95% or more, may be 98% or more, or may be 99% or more based on the number of cells.

The "highly expressing HHEX" is not particularly limited, but means that, for example, when the mRNA expression level in a control fibroblast is assumed to be 1 in the measurement of the mRNA expression level with RT-qPCR, the mRNA expression level of HHEX in the fibroblast in the pharmaceutical composition of the present embodiment is preferably increased by 1.20 times or more, 1.40 times or more, 1.50 times or more, 1.60 times or more, 1.70 times or more, 1.80 times or more, 1.90 times or more, 2.00 times or more, 2.58 times or more, 3.00 times or more, 4.00 times or more, 5.00 times or more, 6.00 times or more, 7.00 times or more, 8.00 times or more, 9.00 times or more, 10.0 times or more, or more times.

The mRNA expression level in a control fibroblast may be the mRNA expression level in an untreated fibroblast or may be the average mRNA expression level in the entire fibroblast after treatment.

When the protein expression level is measured by a flow cytometry method, the proportion of a HHEX-positive (hereinafter, also referred to as "HHEX+") fibroblast may be 0.03% or more, may be 0.1% or more, may be 1% or more, may be 2% or more, may be 4% or more, may be 5% or more, may be 10% or more, may be 20% or more, may be 30% or more, may be 40% or more, may be 50% or more, may be 60% or more, may be 70% or more, may be 80% or more, may be 90% or more, may be 95% or more, may be 98% or more, or may be 99% or more based on the number of cells.

In the present embodiment, the lymphangiogenesis promoting factor preferably means a gene or protein of ADM or HHEX.

The base sequence and the amino acid sequence of the lymphangiogenesis promoting factor are not particularly limited. For example, ADM may be a gene containing a base sequence represented by SEQ ID NO: 1 or a gene containing a base sequence being 90% or more, 95% or more or 98% or more identical to the base sequence, the gene not only being hybridized with a complementary sequence to all or part of the base sequence represented by SEQ ID NO: 1, under stringent conditions, but also encoding a protein having lymphangiogenesis ability. Also, HHEX may be a gene containing a base sequence represented by SEQ ID NO: 2 or a gene containing a base sequence being 90% or more, 95% or more, or 98% or more identical to the base sequence, the gene not only being hybridized with a complementary sequence to all or part of the base sequence represented by SEQ ID NO: 2, under stringent conditions, but also encoding a protein having lymphangiogenesis ability.

The fibroblast in the pharmaceutical composition of the present embodiment may express CD 106 as a cell surface marker.

The fibroblast in the pharmaceutical composition of the present embodiment may be a cell population of fibroblast including the above fibroblast highly expressing ADM and/or HHEX.

In the present embodiment, the proportion of a CD106-positive cell in a cell population of fibroblast including a fibroblast produced by a production method of the fibroblast highly expressing ADM and/or HHEX, described below, may be high, but its reverse may not be necessarily established.

### (Preparation of fibroblast)

The derivation of the fibroblast is not particularly limited in preparation of the fibroblast, and is as described above. In this regard, the fibroblast may be an autologous cell, and, for example, a cardiac fibroblast isolated from a cardiac tissue of a patient suffering from a cardiac disease or a kidney fibroblast differentiated from an adult (somatic) stem cell of a patient and isolated is prepared. A fibroblast differentiated from a pluripotent stem cell such as autologous cell-derived iPS cell and collected may also be adopted. Additionally, a cell other than an autologous cell may also be adopted, and, for example, a cardiac tissue derived from a donor providing a cardiac cell, a cardiac fibroblast isolated from a cardiac tissue produced by use of an animal or the like, or a cardiac fibroblast differentiated from an adult (somatic) stem cell of a donor and isolated is prepared. A fibroblast differentiated from a pluripotent stem cell derived from a donor, such as an iPS cell or an ES cell, and collected may also be adopted.

### (Separation of fibroblast)

The fibroblast prepared can be typically treated with an enzyme such as dispase or collagenase and thus separated. The separation may be performed with an enzyme such as dispase or collagenase, or may be performed by other treatment, for example, mechanical treatment as long as required separation can be made.

### (Method for producing fibroblast highly expressing ADM and/or HHEX)

The method includes contacting TNF-α and IL-4 with a fibroblast, and culturing the fibroblast contacted, under a condition where ADM and/or HHEX are/is capable of being highly expressed.

The method for contacting TNF-α and IL-4 with a fibroblast is not especially limited as long as induction of the fibroblast highly expressing ADM and/or HHEX is not remarkably impaired, and is typically the addition of TNF-α and IL-4 to a medium containing a fibroblast, but not limited thereto. TNF-α and IL-4 may be each added singly, or in combination of a plurality thereof. In the case of a plurality of kinds, TNF-α and IL-4 may be each separately contacted with a fibroblast, or may be contacted therewith at the same time. For example, TNF-α and IL-4 may be dissolved in water, a medium, a serum, or dimethylsulfoxide (DMSO) and then added to a medium.

The fibroblast can be cultured in a medium where TNF-α and IL-4 are added, and thus the fibroblast can be cultured with the expression level(s) of ADM and/or HHEX in the fibroblast being kept. Therefore, a fibroblast having a high expression level(s) of ADM and/or HHEX can be produced.

When TNF-α and IL-4 are added to a medium (mL), the amount of TNF-α added is not especially limited, is usually 0.1 ng/mL or more, and may be 0.5 ng/mL or more, may be 1 ng/mL or more, may be 10 ng/mL or more, or may be 50 ng/mL or more. The upper limit is not particularly limited, is usually 500 ng/mL or less, and may be 100 ng/mL or less.

The amount of IL-4 added is not especially limited, is usually 0.1 ng/mL or more, and may be 0.5 ng/mL or more, may be 1 ng/mL or more, or may be 2 ng/mL or more. The upper limit is not particularly limited, is usually 10 ng/mL or less, and may be 5 ng/mL or less, or may be 1 ng/mL or less.

The ratio (weight ratio) of TNF-α and IL-4 added, TNF-α: IL-4, is usually 10000:1 to 1:1, and may be 50000:1 to 10:1. The ratio is 1:1 to 1:10000, and may be 1:10 to 1:50000.

The fibroblast contacted with TNF-α and IL-4 can be further cultured to thereby produce an ADM and/or HHEX+ fibroblast.

The culturing of the fibroblast is not especially limited as long as it is under a condition where the fibroblast is capable of being ADM and/or HHEX-positive, and may be performed by a known cell culturing method.

The medium used in the culturing can be appropriately set depending on the type and the like of a cell to be cultured, and, for example, DMEM, α-MEM, RPMI-1640, and HFDM-1(+) can be used. A nutritional substance such as FCS or FBS, a growth factor, a cytokine, an antibiotic substance, and/or the like may be added to the medium. Furthermore, the culturing may also be performed in a medium containing TNF-α and IL-4.

The culturing period can be appropriately set with the culturing time and the number of culturing days, depending on the purpose(s) of, for example, allowing the number of cells cultured, to achieve the desired number of cells, and/or allowing a cell cultured, to achieve the desired function. The culturing period is, for example, 1 hour, 2 hours, 3 hours, 4 hours, 5 hours, 6 hours, 7 hours, 8 hours, 9 hours, 10 hours, 11 hours, 12 hours, 15 hours, 18 hours, 21 hours, 1 day, 2 days, 3 days, 4 days, 5 days, 6 days, 7 days, 8 days, 9 days, 10 days, 2 weeks, 1 month, 2 months, 3 months, or 6 months.

The culturing temperature can be appropriately set depending on the type and the like of a cell to be cultured, and may be, for example, 10°C or more, 15°C or more, 20°C or more, 25°C or more, or 30°C or more, and may be 60°C or less, 55°C or less, 50°C or less, 45°C or less, 40°C or less.

The culturing may be performed multiple times. For example, the purity of the fibroblast can be enhanced to the desired value by selection or concentration, and the culturing can be performed in each case.

The production of the fibroblast may further include selecting or concentrating a fibroblast highly expressing ADM and/or HHEX by use of an anti-CD106 antibody. The selection or concentration method is not particularly limited, can be performed by a method known by those skilled in the art, and can be carried out by, for example, performing primary immunostaining with human CD106 (VCAM-1)-biotin (Miltenyi Biotec) and secondary immunostaining with anti-biotin microbeads (Miltenyi Biotec), and collecting a stained cell by autoMACS (Miltenyi Biotec). The selection or concentration may be carried out at any timing.

The anti-CD106 antibody here used can be any known one or can be a commercially available product acquired.

The anti-CD106 antibody can be used for the selection or concentration, resulting in an increase in proportion of a CD106-positive fibroblast and furthermore an increase in proportion of the ADM and/or HHEX+ fibroblast, in a cell population of fibroblast.

The fibroblast cultured may be collected by, for example, detaching the cell with protease such as trypsin, detaching the cell by the change in temperature by use of a temperature responsive culture dish, or detaching the cell by use of a device such as a cell scraper.

The pharmaceutical composition of the present embodiment can be administered or transplanted *in vivo,* to thereby promote lymphangiogenesis *in vivo.* Therefore, for example, a condition of lymphangiogenesis ability reduced due to organ tissue disorder, defect, and/or dysfunction can be ameliorated, a disorder of homeostasis in a tissue fluid or a disorder of transportability of lipid and/or vitamin can be ameliorated, and an immunological surveillance mechanism can be activated, without any particular limitation. The pharmaceutical composition may be one which can be administered to a patient having organ failure, and thus may be one which can treat such organ failure.

The organ failure refers to a condition where no organ performs any function expected and/or organ insufficiency at a level which cannot allow normal homeostasis to be kept without clinical intervention. A specific organ is not particularly limited, and examples thereof include heart, kidney, adrenal gland, thyroid, parathyroid, vocal cords, thymus, cerebrum, cerebellum, brainstem, spinal cord, peripheral nerve, skin, muscle, eyeball, tongue, esophagus, stomach, gallbladder, bile duct, pancreas, pancreas duct, liver, spleen, small intestine, large intestine, rectum, anus, trachea, bronchi, lung, bladder, urinary duct, prostate, artery, vein, lymphatic vessel, lymphatic node, bone, bone marrow, cartilage, tendon, teeth, testicle, deferent duct, penis, uterus, ovary, fallopian tube, and vagina. For example, the organ failure in the heart or the kidney may be a condition where an organ function is reduced as compared with a normal condition, and may be particularly a condition where an organ function is reduced to 30% or less as compared with a normal condition.

The pharmaceutical composition of the present embodiment can be administered or transplanted *in vivo,* to thereby treat or ameliorate fibrosis. The site of onset of fibrosis is not particularly limited, and examples thereof include heart, lung, and kidney.

The method for administering or transplanting the pharmaceutical composition of the present embodiment, to a living body, is not particularly limited, and the pharmaceutical composition can be administered by, for example, injection. The pharmaceutical composition can be directly administered or transplanted to a failed organ or its infarcted peripheral region (for example, subcapsule), or an injured region in an organ where fibrosis is developed, or a fibrotic region or its peripheral region, thereby allowing for amelioration of the disease condition or treatment of organ failure. The pharmaceutical composition may be administered intravenously, intraarterially, to the lymphatic node, to the lymphatic vessel, to the bone marrow, subcutaneously, to the cavernous body, intraperitoneally, intramuscularly, intraspinally, or to the joint cavity. Such intravenous administration, intraarterial administration, or administration to the lymphatic vessel may be performed by injection to the vessel, infusion by a catheter, or any other known procedure.

The injection method is not especially limited, a known injection procedure such as needle injection or needle-free injection can be applied, and the catheter method used in infusion, here applied, can also be a known method, without any particular limitations.

The pharmaceutical composition of the present embodiment may be applied as an artificial organ material to a living body, without any particular limitation, and, for example, the artificial organ material may be any cell constructed as a planar or three-dimensional cellular tissue. The planar or three-dimensional cellular tissue may be one obtained by culturing the fibroblast singly, or may be a planar or three-dimensional cellular tissue obtained by co-culturing the fibroblast with other cell, for example, a kidney cell. The planar or three-dimensional cellular tissue is not particularly limited, and is, for example, a cell sheet, a cell fiber, or a tissue formed by a 3D printer.

In the present embodiment, the size and the shape of the planar or three-dimensional cellular tissue are not particularly limited as long as the cellular tissue can be applied to a necrotized, damaged or fibrotic organ tissue and/or its peripheral region and/or lesion region. The "planar" may be either monolayer or multilayer.

The conditions of cell culturing, including the medium composition, the temperature, and the humidity, are not particularly limited as long as the ADM and/or HHEX+ fibroblast can be cultured and the planar or three-dimensional cellular tissue can be constructed.

The planar or three-dimensional cellular tissue can be applied to a necrotized, damaged or fibrotic organ tissue and/or its peripheral region, or can be exchanged as an artificial organ with a necrotized, damaged or fibrotic organ tissue and/or its peripheral region, to thereby ameliorate a condition of lymphangiogenesis ability reduced, ameliorate a disorder of homeostasis in a tissue fluid or a disorder of transportability of lipid and/or vitamin, activate an immunological surveillance mechanism, treat organ failure, and/or treat or ameliorate fibrosis.

The proportion of the ADM and/or HHEX+ fibroblast in the pharmaceutical composition is appropriately set based on a mode of administration or transplantation to a patient, and a therapeutically effective amount of the ADM and/or HHEX+ fibroblast may be contained as an active ingredient.

When other fibroblast is contained in the pharmaceutical composition, for example, the proportion of the ADM and/or HHEX+ fibroblast relative to the amount of the total fibroblast contained in the pharmaceutical composition, based on the number of cells, may be 0.03% or more, may be 0.1% or more, may be 1% or more, may be 2% or more, may be 4% or more, may be 5% or more, may be 10% or more, may be 20% or more, may be 30% or more, may be 40% or more, may be 50% or more, may be 60% or more, may be 70% or more, may be 80% or more, may be 90% or more, may be 95% or more, may be 98% or more, or may be 99% or more.

When a human-derived fibroblast is used, the ADM and/or HHEX expression level in the ADM and/or HHEX+ fibroblast relative to the ADM and/or HHEX expression level in a control fibroblast, at the number of ADM and/or HHEX+ fibroblasts in the pharmaceutical composition, may be 1.20 times or more, 1.40 times or more, 1.50 times or more, 1.60 times or more, 1.70 times or more, 1.80 times or more, 1.90 times or more, 2.00 times or more, 3.00 times or more, 4.00 times or more, 5.00 times or more, 6.00 times or more, 7.00 times or more, 8.00 times or more, 9.00 times or more, or 10.0 times or more.

The number of ADM and/or HHEX+ fibroblasts in the pharmaceutical composition can be, for example, 1.0 × 10² cells/mL or more, 1.0 × 10³ cells/mL or more, 1.0 × 10⁴ cells/mL or more, 1.0 × 10⁵ cells/mL or more, 1.0 × 10⁶ cells/mL or more, 5.0 × 10⁶ cells/mL or more, or 1.0 × 10⁷ cells/mL or more. The number of CD106-positive fibroblasts in the injectable composition may be further increased or decreased depending on the degree of disease.

The method for confirming the effect of the treating and/or the amelioration by the pharmaceutical composition is not particularly limited, and examples include an increase in lymphangiogenesis after administration or transplantation of the composition as compared with before administration or transplantation of the composition, repair of the functionality of an organ after administration or transplantation of the composition as compared with before administration or transplantation of the composition, and reductions in the degree of damage and degree of fibrosis of an organ after administration or transplantation of the composition as compared with before administration or transplantation of the composition.

The pharmaceutical composition may contain any other ingredient which is physiologically acceptable in the pharmaceutical composition. Examples of such other ingredient include physiological saline, a cell storage solution, a cell culture fluid, hydrogel, an extracellular matrix, and a cryopreserved solution.

Another embodiment of the first invention can provide a method for treating or ameliorating fibrosis by administration or transplantation of the pharmaceutical composition including the cell highly expressing ADM and/or HHEX, to a fibrotic tissue and/or its peripheral region.

Another embodiment of the first invention can provide a method involving administering or transplanting the pharmaceutical composition including the cell highly expressing ADM and/or HHEX, to a failed organ tissue and/or its peripheral region, to thereby ameliorate a condition of lymphangiogenesis ability reduced, ameliorate a disorder of homeostasis in a tissue fluid, reduce edema, ameliorate a disorder of transportability of lipid and/or vitamin, activate an immunological surveillance mechanism, and/or treat organ failure.

Another embodiment of the first invention can provide use of the composition including the ADM and/or HHEX+ cell, as an injection or a transplantation material for administration or transplantation to a failed organ tissue and/or its peripheral region, or a fibrotic tissue and/or its peripheral region.

### <Fibroblast highly expressing ADM>

One embodiment of the second invention provides a fibroblast highly expressing ADM. The fibroblast of the present embodiment is preferably CD106-positive. Furthermore, the fibroblast may highly express VEGF-C.

The derivation of the fibroblast is not restricted, and a pluripotent stem cell such as an embryonic stem cell (ES cell), an induced pluripotent stem cell (iPS cell) and a Muse cell, or an adult stem cell such as a mesenchymal stem cell may be differentiated and used. A primary cell collected from an animal (including human) may be used, and an established cell may also be used.

The fibroblast may be derived from an organ, such as lung, heart, kidney, or skin.

The fibroblast is preferably adult-derived without any particular limitation.

A fibroblast from which the fibroblast of the present embodiment is derived may originally express VEGF-C or ADM at a certain level, or may not express VEGF-C or ADM at all.

The fibroblast in the present embodiment may be a cell expressing a cell surface marker expressed by a usual fibroblast, or furthermore may be a cell additionally expressing other markers or not expressing some markers by the cell production method of the present invention described below.

The "highly expressing VEGF-C" is not particularly limited, but means that, for example, when the mRNA expression level in a control fibroblast is assumed to be 1 in measurement of the mRNA expression level with RT-qPCR, the mRNA expression level of VEGF-C in the fibroblast in the pharmaceutical composition of the present embodiment is preferably increased by 1.20 times or more, 1.40 times or more, 1.50 times or more, 1.60 times or more, 1.70 times or more, 1.80 times or more, 1.90 times or more, 2.00 times or more, 2.60 times or more, 2.99 times or more, 3.00 times or more, 3.38 times or more, 3.52 times or more, 4.00 times or more, 4.11 times or more, 4.70 times or more, 5.00 times or more, 6.00 times or more, 7.00 times or more, 8.00 times or more, 9.00 times or more, 10.0 times or more, or more times.

The mRNA expression level in a control fibroblast may be the mRNA expression level in an untreated fibroblast or may be the average mRNA expression level in the entire fibroblast after treatment.

When the protein expression level is measured by a flow cytometry method, the proportion of a VEGF-C-positive (hereinafter, also referred to as "VEGF-C+") fibroblast may be 0.03% or more, may be 0.1% or more, may be 1% or more, may be 2% or more, may be 4% or more, may be 5% or more, may be 10% or more, may be 20% or more, may be 30% or more, may be 40% or more, may be 50% or more, may be 60% or more, may be 70% or more, may be 80% or more, may be 90% or more, may be 95% or more, may be 98% or more, or may be 99% or more based on the number of cells.

The "highly expressing ADM" is not particularly limited, but means that, for example, when the mRNA expression level in a control fibroblast is assumed to be 1 in measurement of the mRNA expression level with RT-qPCR, the mRNA expression level of ADM in the fibroblast in the pharmaceutical composition of the present embodiment is preferably increased by 1.20 times or more, 1.40 times or more, 1.43 times or more, 1.50 times or more, 1.60 times or more, 1.70 times or more, 1.80 times or more, 1.90 times or more, 1.91 times or more, 1.97 times or more, 2.00 times or more, 2.46 times or more, 3.00 times or more, 3.15 times or more, 4.00 times or more, 5.00 times or more, 6.00 times or more, 7.00 times or more, 8.00 times or more, 9.00 times or more, 10.0 times or more, or more times.

The mRNA expression level in a control fibroblast may be the mRNA expression level in an untreated fibroblast or may be the average mRNA expression level in the entire fibroblast after treatment.

When the protein expression level is measured by a flow cytometry method, the proportion of an ADM-positive (hereinafter, also referred to as "ADM+") fibroblast may be 0.1% or more, may be 1% or more, may be 2% or more, may be 4% or more, may be 5% or more, may be 10% or more, may be 20% or more, may be 30% or more, may be 40% or more, may be 50% or more, may be 60% or more, may be 70% or more, may be 80% or more, may be 90% or more, may be 95% or more, may be 98% or more, or may be 99% or more based on the number of cells.

In the present embodiment, the base sequence and the amino acid sequence of VEGF-C and ADM are not particularly limited. For example, VEGF-C may be a gene containing a base sequence represented by SEQ ID NO: 13 or 14 or a gene containing a base sequence being 90% or more, 95% or more or 98% or more identical to the base sequence, the gene not only being hybridized with a complementary sequence to all or part of the base sequence represented by SEQ ID NO: 13 or 14, under stringent conditions, but also encoding a protein having ability of VEGF-C. Also, ADM may be a gene containing a base sequence represented by SEQ ID NO: 1 or 15 or a gene containing a base sequence being 90% or more, 95% or more, or 98% or more identical to the base sequence, the gene not only being hybridized with a complementary sequence to all or part of the base sequence represented by SEQ ID NO: 1 or 15, under stringent conditions, but also encoding a protein having ability of ADM.

The fibroblast of the present embodiment preferably expresses CD 106 as a cell surface marker.

The present embodiment may provide a cell population of fibroblast including the above fibroblast highly expressing ADM.

### (Preparation of fibroblast)

The derivation of the fibroblast is not particularly limited in preparation of the fibroblast, and is as described above. In this regard, the fibroblast may be an autologous cell, and, for example, a pulmonary fibroblast isolated from a lung tissue of a patient suffering from a pulmonary disease or a pulmonary fibroblast differentiated from an adult (somatic) stem cell of a patient and isolated may be prepared. A fibroblast differentiated from a pluripotent stem cell such as autologous cell-derived iPS cell and collected may also be adopted. Additionally, a cell other than an autologous cell may also be adopted, and, for example, a lung tissue derived from a donor providing a lung cell, a pulmonary fibroblast isolated from a lung tissue produced by use of an animal or the like, or a pulmonary fibroblast differentiated from an adult (somatic) stem cell of a donor and isolated may be prepared. Alternatively, a fibroblast differentiated from a pluripotent stem cell derived from a donor, such as an iPS cell or an ES cell, and collected may also be adopted.

### (Separation of fibroblast)

The fibroblast prepared can be typically treated with an enzyme such as dispase or collagenase and thus separated. The separation may be performed by an enzyme such as dispase or collagenase, or may be performed by other treatment, for example, mechanical treatment as long as required separation can be made.

### (Culturing of fibroblast)

The fibroblast may be contacted with TNF-α and IL-4 by a method described below, and may be subjected to culturing for the purpose(s) of, for example, allowing the number of cells to achieve the desired number of cells and/or allowing the desired function to be achieved. The culturing conditions are not restricted, and the culturing may be carried out by a known cell culturing method.

The medium used in the culturing can be appropriately set depending on the type and the like of a cell to be cultured, and, for example, HFDM-1(+), DMEM, α-MEM, and RPMI-1640 can be used. A nutritional substance such as NBCS, FCS or FBS, a growth factor, a cytokine, an antibiotic substance, and/or the like may be added to the medium. Furthermore, the culturing may also be performed in a medium containing TNF-α and IL-4.

The culturing period can be appropriately set with the culturing time and the number of culturing days, depending on the purpose(s) of, for example, allowing the number of cells cultured, to achieve the desired number of cells, and/or allowing a cell cultured, to achieve the desired function. The culturing period is, for example, 1 hour, 2 hours, 3 hours, 4 hours, 5 hours, 6 hours, 7 hours, 8 hours, 9 hours, 10 hours, 11 hours, 12 hours, 15 hours, 18 hours, 21 hours, 1 day, 2 days, 3 days, 4 days, 5 days, 6 days, 7 days, 8 days, 9 days, 10 days, 2 weeks, 1 month, 2 months, 3 months, or 6 months.

The culturing temperature can be appropriately set depending on the type and the like of a cell to be cultured, and may be, for example, 10°C or more, 15°C or more, 20°C or more, 25°C or more, or 30°C or more, and may be 60°C or less, 55°C or less, 50°C or less, 45°C or less, or 40°C or less.

The culturing may be performed multiple times. For example, the purity of the desired fibroblast can be enhanced by selection or concentration, and the culturing can be performed in each case.

### (Collection of fibroblast)

The fibroblast cultured may be collected by detaching the cell with protease such as trypsin, detaching the cell by the change in temperature by use of a temperature responsive culture dish, or detaching the cell by use of a device such as a cell scraper.

Another embodiment of the second invention provides a method for producing a fibroblast highly expressing ADM, the method including contacting TNF-α and IL-4 with a fibroblast, and culturing the fibroblast contacted, under a condition where ADM is capable of being highly expressed. The fibroblast of the present embodiment is preferably CD106-positive. Furthermore, the fibroblast may highly express VEGF-C.

The method for contacting TNF-α and IL-4 with a fibroblast is not especially limited as long as induction of the fibroblast highly expressing ADM, or induction of a fibroblast which highly expresses ADM and which highly expresses VEGF-C and/or which is CD106-positive is not remarkably impaired, and is typically the addition of TNF-α and IL-4 to a medium containing a fibroblast, but not limited thereto. TNF-α and IL-4 may be each separately contacted with a fibroblast, or may be contacted therewith at the same time. For example, TNF-α and IL-4 may be dissolved in water, a medium, a serum, or dimethylsulfoxide (DMSO) and then added to a medium.

In the present embodiment, the fibroblast can be cultured in a medium where TNF-α and IL-4 are added, and thus the fibroblast can be cultured with the expression level of ADM in the fibroblast being kept and furthermore the expression level of VEGF-C being kept. Thus, a fibroblast having a high ADM expression level and furthermore a high VEGF-C expression level can be produced.

When TNF-α and IL-4 are added to a medium (mL), the amount of TNF-α added is not especially limited, is usually 0.1 ng/mL or more, and may be 0.5 ng/mL or more, may be 1 ng/mL or more, may be 10 ng/mL or more, or may be 50 ng/mL or more. The upper limit is not particularly limited, is usually 500 ng/mL or less, and may be 100 ng/mL or less.

The amount of IL-4 added is not especially limited, is usually 0.1 ng/mL or more, may be 0.5 ng/mL or more, may be 1 ng/mL or more, or may be 2 ng/mL or more. The upper limit is not particularly limited, is usually 10 ng/mL or less, may be 5 ng/mL or less, or may be 2 ng/mL or less.

The ratio (weight ratio) of TNF-α and IL-4 added, TNF-α: IL-4, is usually 10000:1 to 1:1, and may be 50000:1 to 10:1. The ratio is 1:1 to 1:10000, and may be 1:10 to 1:50000.

The fibroblast contacted with TNF-α and IL-4 can be further cultured to thereby produce a fibroblast highly expressing ADM. The fibroblast may also highly express VEGF-C.

The culturing of the fibroblast is not especially limited as long as it is under a condition where the fibroblast is capable of highly expressing ADM, and may be performed by a known cell culturing method. The culturing may be under a condition where VEGF-C is capable of being highly expressed, and is preferably under a condition where CD106-positive can be achieved. The method described in the section (Culturing of fibroblast) in the fibroblast production method is exemplified.

When the fibroblast in the present embodiment is CD106-positive, a CD106-positive fibroblast highly expressing ADM can be selected or concentrated by use of an anti-CD106 antibody. Furthermore, the fibroblast may highly express VEGF-C. The selection or concentration method is not particularly limited, can be performed by a method known by those skilled in the art, and can be carried out by, for example, performing primary immunostaining with human CD106 (VCAM-1)-biotin (Miltenyi Biotec) and secondary immunostaining with anti-biotin microbeads (Miltenyi Biotec), and collecting a stained cell by autoMACS (Miltenyi Biotec). The selection or concentration may be carried out at any timing.

The anti-CD106 antibody here used can be any known one or can be a commercially available product acquired.

The anti-CD106 antibody can be used for the selection or concentration, resulting in an increase in proportion of the fibroblast highly expressing ADM in the cell population of fibroblast.

The collection of the fibroblast cultured is, for example, the method described in the section (Collection of fibroblast) in the fibroblast production method.

The fibroblast highly expressing ADM is obtained by the method for producing the fibroblast highly expressing ADM of the present embodiment, and another embodiment of the second invention provides the fibroblast or a cell population of fibroblast including the fibroblast. Furthermore, the fibroblast may highly express VEGF-C or may be CD106-positive. The fibroblast is stimulated by TNF-α and IL-4 and thus increased in expression levels of ADM and VEGF-C, and thus the fibroblast highly expressing ADM and furthermore the fibroblast highly expressing VEGF-C can be suitably used in a therapeutic composition to a disease causing deterioration in lymphangiogenesis ability and a pulmonary disease such as pulmonary fibrosis or interstitial pneumonia. In the present embodiment, the derivation of the fibroblast is not particularly limited, and the fibroblast is preferably lung-derived.

Another embodiment may provide a cell population of fibroblast including a fibroblast obtained from the fibroblast highly expressing ADM.

### <Cell population including CD 106-positive pulmonary fibroblast>

Another embodiment of the second invention provides a cell population including a CD106-positive pulmonary fibroblast. The fibroblast of the present embodiment may highly express ADM, or may highly express VEGF-C.

The derivation of the fibroblast is not restricted, and a pluripotent stem cell such as an embryonic stem cell (ES cell), an induced pluripotent stem cell (iPS cell) and a Muse cell, or an adult stem cell such as a mesenchymal stem cell may be differentiated and used. A primary cell collected from an animal (including human) may be used, and an established cell may also be used.

The fibroblast is not particularly limited, and is preferably adult-derived.

A fibroblast from which the fibroblast of the present embodiment is derived may originally express VEGF-C or ADM at a certain level, or may not express VEGF-C or ADM at all.

The fibroblast in the present embodiment may be a cell expressing a cell surface marker expressed by a usual fibroblast, or furthermore may be a cell additionally expressing other markers or not expressing some markers by the cell production method of the present invention described below.

The proportion of the CD106-positive fibroblast relative to the total fibroblast in the cell population of fibroblast is not especially limited, and may be more than 18.01%, may be more than 27.97%, may be more than 30%, may be more than 31.38%, may be more than 34.79%, may be more than 40%, may be more than 50%, may be more than 60%, may be more than 70%, may be more than 80%, may be more than 90%, may be more than 95%, or may be more than 97.10% based on the number of cells. The proportion may be 18.01% or more, may be 27.97% or more, may be 30% or more, may be 31.38% or more, may be 34.79% or more, may be 40% or more, may be 50% or more, may be 60% or more, may be 70% or more, may be 80% or more, may be 90% or more, may be 95% or more, or may be 97.10% or more based on the number of cells.

The cell population of fibroblast of the present embodiment may be a cell population of CD90-positive fibroblast. The proportion of the CD106-positive and CD90-positive fibroblast relative to the total fibroblast in the cell population of fibroblast is not especially limited, and may be more than 17.97%, may be more than 27.91%, may be more than 30%, may be more than 31.21%, may be more than 34.51%, may be more than 40%, may be more than 50%, may be more than 60%, may be more than 70%, may be more than 80%, may be more than 90%, may be more than 95%, or may be more than 96.29% based on the number of cells. The proportion may be 17.97% or more, may be 27.91% or more, may be 30% or more, may be 31.21% or more, may be 34.51% or more, may be 40% or more, may be 50% or more, may be 60% or more, may be 70% or more, may be 80% or more, may be 90% or more, may be 95% or more, or may be 96.29% or more based on the number of cells.

The description in the above section <Fibroblast highly expressing ADM> can be employed for the "highly expressing ADM" and the "highly expressing VEGF-C".

### (Preparation of fibroblast)

The derivation of the fibroblast is not particularly limited in preparation of the fibroblast, and is as described above. In this regard, the fibroblast may be an autologous cell, and, for example, a pulmonary fibroblast isolated from a lung tissue of a patient suffering from a pulmonary disease or a pulmonary fibroblast differentiated from an adult (somatic) stem cell of a patient and isolated may be prepared. A fibroblast differentiated from a pluripotent stem cell such as autologous cell-derived iPS cell and collected may also be adopted. Additionally, a cell other than an autologous cell may also be adopted, and, for example, a lung tissue derived from a donor providing a lung cell, a pulmonary fibroblast isolated from a lung tissue produced by use of an animal or the like, or a pulmonary fibroblast differentiated from an adult (somatic) stem cell of a donor and isolated may be prepared. Alternatively, a fibroblast differentiated from a pluripotent stem cell derived from a donor, such as an iPS cell or an ES cell, and collected may also be adopted.

### (Separation of fibroblast)

The fibroblast prepared can be typically treated with an enzyme such as dispase or collagenase and thus separated. The separation may be performed by an enzyme such as dispase or collagenase, or may be performed by other treatment, for example, mechanical treatment as long as required separation can be made.

### (Culturing of fibroblast)

The fibroblast may be contacted with TNF-α and IL-4 by a method described below, and may be subjected to culturing for the purpose(s) of, for example, allowing the number of cells to achieve the desired number of cells and/or allowing the desired function to be achieved. The culturing conditions are not restricted, and the culturing may be carried out by a known cell culturing method.

The medium used in the culturing can be appropriately set depending on the type and the like of a cell to be cultured, and, for example, HFDM-1(+), DMEM, α-MEM, and RPMI-1640 can be used. A nutritional substance such as NBCS, FCS or FBS, a growth factor, a cytokine, an antibiotic substance, and/or the like may be added to the medium. Furthermore, the culturing may also be performed in a medium containing TNF-α and IL-4.

The culturing period can be appropriately set with the culturing time and the number of culturing days, depending on the purpose(s) of, for example, allowing the number of cells cultured, to achieve the desired number of cells, and/or allowing a cell cultured, to achieve the desired function. The culturing period is, for example, 1 hour, 2 hours, 3 hours, 4 hours, 5 hours, 6 hours, 7 hours, 8 hours, 9 hours, 10 hours, 11 hours, 12 hours, 15 hours, 18 hours, 21 hours, 1 day, 2 days, 3 days, 4 days, 5 days, 6 days, 7 days, 8 days, 9 days, 10 days, 2 weeks, 1 month, 2 months, 3 months, or 6 months.

The culturing temperature can be appropriately set depending on the type and the like of a cell to be cultured, and may be, for example, 10°C or more, 15°C or more, 20°C or more, 25°C or more, or 30°C or more, and may be 60°C or less, 55°C or less, 50°C or less, 45°C or less, or 40°C or less.

The culturing may be performed multiple times. For example, the purity of the desired fibroblast can be enhanced by selection or concentration, and the culturing can be performed in each case.

### (Collection of fibroblast)

The fibroblast cultured may be collected by detaching the cell with protease such as trypsin, detaching the cell by the change in temperature by use of a temperature responsive culture dish, or detaching the cell by use of a device such as a cell scraper.

### (Cell sorting of CD106-positive fibroblast)

When the fibroblast is CD106-positive, the fibroblast may be subjected to CD106-positive fibroblast selection or concentration. When a CD106-positive fibroblast can be obtained without the selection or concentration, the selection or concentration can be omitted. Examples of the selection or concentration of the CD106-positive fibroblast include methods with an anti-CD106 antibody (anti-VCAM-1 antibody), such as a flow cytometry method, a magnetic bead method, an affinity column method, or a panning method. Specifically, a magnetic cell separation method (MACS), a fluorescent label cell separation method (FACS), or the like can be used. The anti-CD106 antibody here used may be commercially available one or may be produced by a known method. Either a monoclonal antibody or a polyclonal antibody may be used, and a monoclonal antibody is preferably used from the viewpoint of specificity. Furthermore, the CD106-positive fibroblast may be obtained by introducing a drug-resistant gene and excluding a CD106-negative fibroblast. A fluorescent protein-positive cell may be isolated with FACS or the like by introducing a fluorescent protein-encoding gene. A group of expression of a CD106 gene may be isolated by confirming expression of a CD106 gene in a cell by use of a procedure such as real-time PCR, a next-generation sequencer and *in-situ* hybridization.

### <Pharmaceutical composition including fibroblast highly expressing ADM or its cell population, or pharmaceutical composition including cell population of CD106-positive pulmonary fibroblast>

Another embodiment of the second invention provides a pharmaceutical composition including the fibroblast highly expressing ADM or its cell population, or a pharmaceutical composition including a cell population of the CD106-positive pulmonary fibroblast. The fibroblast of the present embodiment is preferably CD106-positive. Furthermore, the fibroblast may highly express VEGF-C.

ADM and VEGF-C are lymphangiogenesis factors, and thus, for example, the pharmaceutical composition of the present embodiment can be administered or transplanted to an organ of a living body, for example, the lung, or its peripheral region to thereby ameliorate a condition of lymphangiogenesis ability reduced in an organ, for example, the lung. Therefore, for example, a condition of lymphangiogenesis ability reduced can be ameliorated in an organ, for example, the lung without any particular limitation. In particular, the pharmaceutical composition is useful in treating a pulmonary disease such as pulmonary fibrosis or interstitial pneumonia. In the present embodiment, the derivation of the fibroblast is not particularly limited, and the fibroblast is preferably lung-derived.

The method for administering or transplanting the pharmaceutical composition of the present embodiment, to a living body, is not particularly limited, and the pharmaceutical composition can be administered by, for example, injection. The pharmaceutical composition can be directly administered or transplanted to an injured region in an organ, for example, the lung, or a fibrotic region or its peripheral region, thereby allowing for amelioration of the above disease condition or treatment of an organ, for example, the lung. The pharmaceutical composition may be administered intravenously, intraarterially, to the lymphatic node, to the lymphatic vessel, to the bone marrow, subcutaneously, to the cavernous body, intraperitoneally, intramuscularly, intraspinally, or to the joint cavity. Such intravenous administration, intraarterial administration, or administration to the lymphatic vessel may be performed by injection to the vessel, infusion by a catheter, or any other known procedure.

The injection method is not especially limited, a known injection procedure such as needle injection or needle-free injection can be applied, and the catheter method used in infusion, here applied, can also be a known method, without any particular limitations.

The pharmaceutical composition of the present embodiment may be applied as an artificial organ material to a living body, without any particular limitation, and, for example, the artificial organ material may be any cell constructed as a planar or three-dimensional cellular tissue. The planar or three-dimensional cellular tissue may be one obtained by culturing the fibroblast singly, or may be a planar or three-dimensional cellular tissue obtained by co-culturing the fibroblast with other cell, for example, a lung cell. The planar or three-dimensional cellular tissue is not particularly limited, and is, for example, a cell sheet, a cell fiber, or a tissue formed by a 3D printer.

In the present embodiment, the size and the shape of the planar or three-dimensional cellular tissue are not particularly limited as long as the cellular tissue can be applied to a damaged or fibrotic lung tissue and/or its peripheral region and/or lesion region. The "planar" may be either monolayer or multilayer.

The conditions of cell culturing, including the medium composition, the temperature, and the humidity, are not particularly limited as long as a CD106-positive fibroblast highly expressing VEGF-C and/or ADM can be cultured and the planar or three-dimensional cellular tissue can be constructed. For example, those according to the culturing conditions described in the section(s) of (Culturing of fibroblast) and/or (Collection of fibroblast) in the fibroblast production method can be exemplified.

The planar or three-dimensional cellular tissue can be applied to a damaged or fibrotic organ tissue and/or its peripheral region, or can be exchanged as an artificial organ with a damaged or fibrotic organ tissue and/or its peripheral region, to thereby ameliorate a condition of lymphangiogenesis ability reduced, and/or treat a pulmonary disease such as pulmonary fibrosis or interstitial pneumonia.

The proportion of the fibroblast highly expressing ADM in the pharmaceutical composition is appropriately set based on a mode of administration or transplantation to a patient, and a therapeutically effective amount of the fibroblast highly expressing ADM may be contained as an active ingredient.

When other fibroblast is contained in the pharmaceutical composition, for example, the proportion of the fibroblast highly expressing ADM relative to the amount of the total fibroblast contained in the pharmaceutical composition may be 0.03% or more, may be 0.1% or more, may be 1% or more, may be 2% or more, may be 4% or more, may be 5% or more, may be 10% or more, may be 20% or more, may be 30% or more, may be 40% or more, may be 50% or more, may be 60% or more, may be 70% or more, may be 80% or more, may be 90% or more, may be 95% or more, may be 98% or more, or may be 99% or more based on the number of cells.

The proportion of the CD106-positive pulmonary fibroblast in the pharmaceutical composition is appropriately set based on a mode of administration or transplantation to a patient, and a therapeutically effective amount of the fibroblast highly expressing ADM may be contained as an active ingredient. Specifically, the proportion of the CD 106-positive pulmonary fibroblast described in the section <Cell population including CD106-positive pulmonary fibroblast> can be employed.

The number of fibroblasts highly expressing ADM or CD106-positive pulmonary fibroblasts in the pharmaceutical composition can be, for example, 1.0 × 10² cells/mL or more, 1.0 × 10³ cells/mL or more, 1.0 × 10⁴ cells/mL or more, 1.0 × 10⁵ cells/mL or more, 1.0 × 10⁶ cells/mL or more, 5.0 × 10⁶ cells/mL or more, or 1.0 × 10⁷ cells/mL or more. The number of cells in the injectable composition may be further increased or decreased depending on the degree of disease.

The method for confirming the effect of the treating and/or the amelioration by the pharmaceutical composition is not particularly limited, and examples include an increase in lymphangiogenesis after administration or transplantation of the composition as compared with before administration or transplantation of the composition, repair of the functionality of an organ, for example, the lung, after administration or transplantation of the composition as compared with before administration or transplantation of the composition, and reductions in the degree of damage and degree of fibrosis of an organ, for example, the lung, after administration or transplantation of the composition as compared with before administration or transplantation of the composition.

The pharmaceutical composition may contain any other ingredient which is physiologically acceptable in the pharmaceutical composition. Examples of such other ingredient include physiological saline, a cell storage solution, a cell culture fluid, hydrogel, an extracellular matrix, and a cryopreserved solution.

Another embodiment of the second invention can provide a method for treating a pulmonary disease such as pulmonary fibrosis or interstitial pneumonia by administering or transplanting the fibroblast highly expressing ADM, its cell population, a cell population of the CD106-positive pulmonary fibroblast, or a pharmaceutical composition including the same, to an organ tissue, for example, the lung tissue and/or its peripheral region. The fibroblast of the present embodiment is preferably CD106-positive. Furthermore, the fibroblast may highly express VEGF-C.

Another embodiment of the second invention can provide a method for ameliorating a condition of lymphangiogenesis ability reduced, by administering or transplanting the fibroblast highly expressing ADM, its cell population, a cell population of the CD106-positive pulmonary fibroblast, or a pharmaceutical composition including the same, to an organ tissue, for example, the lung tissue and/or its peripheral region. The fibroblast of the present embodiment is preferably CD106-positive. Furthermore, the fibroblast may highly express VEGF-C.

Another embodiment of the second invention can provide use of the fibroblast highly expressing ADM, its cell population or a composition including the same, as an injection or a transplantation material for administration or transplantation to an organ tissue, for example, the lung tissue and/or its peripheral region. The fibroblast of the present embodiment is preferably CD106-positive. Furthermore, the fibroblast may highly express VEGF-C.

### Examples

Hereinafter, the present invention will be described with reference to Examples in more detail, but the scope of the present invention is, of course, not limited to only such Examples.

### <Example A: Example of pharmaceutical composition including fibroblast highly expressing ADM and/or HHEX>

### <Increase in CD106-positive cell ratio of human adult-derived cardiac fibroblast>

After the heart of a human adult was purchased from Science Care and subjected to enzyme treatment with Liberase MNP-S (Roche), a human adult-derived cardiac fibroblast was isolated. This fibroblast was cultured in a serum-free culture solution (HFDM-1(+), Cell Science & Technology) for recombinant human epidermal growth factor (rh-EGF)-containing fibroblasts, where a 1% newborn calf serum (NBCS, Hyclone) was added. After five passages, culturing was performed in a cell culture dish, and a group of culturing without treatment (NT) and a group of treatment by two-agent mixing of TNF-α (50 ng/mL) (Peprotech) and IL-4 (2 ng/mL) (Wako) (+CKs) were provided. All the groups were subjected to culturing for 1 day (24 hours) with HFDM-1(+) where 1% NBCS was added, as a basic medium.

### <Flow cytometry>

A fibroblast was subjected to immunofluorescent staining with BV421 mouse anti-human CD106 (VCAM1) antibody (BD Biosciences) and human CD90-PE (Miltenyi Biotec, Bergisch Gladbach, Germany). Isotype controls here used were a BV421 mouse IgG1; κ isotype control (BD Biosciences) and an REA control (S)-PE (Miltenyi Biotec). The fibroblast was a living cell and subjected to reaction with an antibody at 4°C for 30 minutes.

The fibroblast subjected to the immunofluorescent staining was analyzed by a flow cytometer (MACSQuant Analyzer 10, Miltenyi Biotec). After a cell region was identified by FSC-A and SSC-A, the positive cell ratio (%, in terms of number of cells) to a CD106 protein was evaluated.

### <Increase in expression of ADM and/or HHEX gene in human adult-derived cardiac fibroblast>

A human adult-derived cardiac fibroblast was cultured in a cell culture dish, and a group of culturing without treatment (NT) and a group of treatment by two-agent mixing of TNF-α (50 ng/mL) (Peprotech) and IL-4 (2 ng/mL) (Wako) (+CKs) were provided. All the groups were subjected to culturing for 1 day (24 hours) with HFDM-1(+) where 1% NBCS was added, as a basic medium.

### <Production of primer and probe>

Sequences (SEQ ID NOs: 4 to 9) of a primer used were designed based on human mRNA sequences (ADM, NM_001124.3 (SEQ ID NO: 1); HHEX, NM_002729.5 (SEQ ID NO: 2); ACTB (β-Actin), NM_001101.5 (SEQ ID NO: 3)) published in NCBI so that the anneal temperature calculated by a nearest neighbor base-pair method was about 60°C and one or more introns were sandwiched. A 5'-nuclease probe sequence was also designed based on the published human mRNA sequence data, and the anneal temperature of the probe was designed to a temperature of 5°C or higher than that of the primer and the binding position of the probe was designed so as to be bound to a base between the binding position of the forward primer and the binding position of the reverse primer on the gene of a measurement subject. A dual quencher probe where a fluorescent dye 6-FAM was bound to the 5'-end of the probe, a quencher dye ZEN was bound at the 3'-side by nine bases from the end and a second quencher dye Iowa Black FQ was bound to the 3'-end was obtained, and a back ground signal in RT-PCR analysis was decreased (SEQ ID NOs: 10 to 12). Synthesis of all the above primers and probes was outsourced to Integrated DNA Technologies (Table 1).

**[Table 1]**

| Table 1. Sequence information of primer and probe | | |
|---|---|---|
| Gene | | Sequence |
| ADM | Forward Primer | 5'-atgtacctgggttcgctcgc-3' (SEQ ID NO:4) |
| | Reverse Primer | 5'-ccacgactcagagcccactt-3' (SEQ ID NO:5) |
| | Probe | SG-FAM/ttcgaaact/ZEN/ccgacgcgacatcc/3IABkFQ(SEQ ID NO: 10) |
| HHEX | Forward Primer | 5'-agcgagagacaggtcaaaac-3' (SEQ ID NO:6) |
| | Reverse Primer | 5'-ctgttcactgggcaaatcttg-3' (SEQ ID NO:7) |
| | Probe | 56-FAM/cctccattt/ZEN/agcgcgtcgattctga/3IABkFQ (SEQ ID NO:11) |
| β-Actin | Forward Primer | 5'-accttctacaatgagctgcg-3' (SEQ ID NO:8) |
| | Reverse Primer | 5'-cctggatagcaacgtacatgg-3' (SEQ ID NO:9) |
| | Probe | 56-FAM/atctgggtc/ZEN/atcttctcgcggttg/3IABkFQ (SEQ ID NO:12) |

### <RNA extraction>

The whole RNA was extracted from a cardiac fibroblast by use of Qiagen RNeasy Plus Mini Kit (QIAGEN). Here, a fibroblast lysate was prepared with a QIA shredder (QIAGEN), and other operations including DNase treatment were made according to a recommended protocol from the kit manufacturer. The concentration of the whole RNA obtained was measured by measuring the absorbance at a wavelength of 260 nm with a Nanodrop One/One spectrophotometer (Thermo Fisher Scientific). The value of A260/A280, representing the degree of contamination of protein, was calculated at the same time, and A260/A280 > 2.0 was confirmed.

### <Real time quantitative RT-PCR>

The reverse transcription reaction and RT-PCR were collectively performed with the same tube by use of One Step PrimeScript 3 RT-qPCR Mix, with UNG Kit (TaKaRa). The reaction was performed in a reaction liquid (total amount 12.5 µL) containing 1 × One Step PrimeScript 3 RT-qPCR Mix, with UNG, 0.2 µM Forward & Reverse primer, a 0.2 µM specific probe, and a 10 ng of a template RNA solution. The reverse transcription reaction was first performed by warming to 52°C for 5 minutes and thereafter heating was made at 95°C for 10 seconds for inactivation of a reverse transcription enzyme. Subsequently, a cycle for heating at 95°C for 5 seconds and at 60°C for 30 seconds was repeated 40 times, and the fluorescence intensity was measured with respect to each of such cycles. A set of reactions and measurements was performed with Thermal Cycler Dice Real Time System 3 (TaKaRa). An RNA solution extracted from a sample confirmed about expression of ADM and VEGF-C was concurrently used as a preparation for creation of a calibration curve, to perform the measurement by use of the RNA solution serially diluted at 4 stages from 1 µg by 10-fold. All the samples were subjected to the measurement at N = 3.

After completion of PCR, the baseline and the threshold of the fluorescence intensity in the resulting amplification curve were set with respect to each target gene, and the number of cycles (Ct value), at which the fluorescent signal reached the threshold, was determined with respect to each of the samples. The analysis was performed with Thermal Cycler Dice Real Time System 3 Software (TaKaRa). Since the correlation coefficient and the amplification efficiency in the calibration curve of each gene were respectively 0.98 or more and 90% or more and the calibration curve was considered to be satisfactory, the Ct value of a subject sample was applied to the calibration curve, and the mRNA content of the objective gene was calculated as a relative value. The relative value was normalized with the value of β-Actin.

### <Lymphatic vessel formation assay>

The lymphatic vessel formation assay was made by co-culturing a human lymphatic endothelial cell collected from a heart-derived cell and any of various human cardiac fibroblasts, by a procedure known by those skilled in the art, and evaluating the cardiac lymphangiogenesis effect by each of various cardiac fibroblasts (Non-Patent Document 15). Specifically, a human lymphatic endothelial cell collected from a human heart-derived cell and any of various human adult-derived cardiac fibroblasts were seeded at a concentration of 2.45 × 10⁵ cells/cm² and at a ratio of 2:8, and co-cultured in EGM-2MV Microvascular Endothelial Cell Growth Medium-2 BulletKit (Lonza, Basel, Switzerland) for 3 days. After the co-culturing, these cells were fixed with 4% paraformaldehyde, subjected to cell membrane permeabilization with 0.1% TritonX, and then subjected to immunofluorescent staining. Primary antibodies here used were an anti-VE-Cadherin rabbit polyclonal antibody (abcam, Cambridge, UK) and an anti-Vimentin mouse polyclonal antibody (abcam). Secondary antibodies here used were goat anti-rabbit IgG H&L (Alexa Fluor 488) (abcam) and goat anti-rabbit mouse IgG H&L (Alexa Fluor 647) (abcam). Nuclear staining was performed with a Hoechst 33258 solution (DOJINDO LABORATORIES., Kumamoto, Japan). A staining sample was imaged with IN Cell Analyzer 2200 (Cytiva, Marlborough, MA).

Quantitative evaluation was performed with Analyzer for ImageJ (v. 1.0.c, Gilles Carpentier, 2012; available online: http://imagej.nih.gov/ij/macros/toolsets/Angiogenesis%20Analyzer.txt). Specifically, a channel of interest was extracted in Icy (v.2.0.3.0; BioImage Analysis Lab, Institut Pasteur), and the background was subjected to a sub-tractor. Thereafter, an image was corrected by application of an anisotropic PDE-based filter (Icy plugin: "Membrane Filter", v.0.1.0.1), the contrast of the image filtered was highlighted and normalized, and the background was subtracted to thereby define ROI. Finally, the resulting image was analyzed with Angiogenesis Analyzer macro. The detailed protocol was published online on dx.doi.org/10.17504/protocols.io.bhtaj6ie.

The entire length and the number of junctions of a mature lymphatic vessel were illustrated, and the quantitative data was represented by Fold increase under the assumption that the average value of NT was 1.

### <Preparation of cell for transplantation>

Respective cells were purchased from the following manufacturers: human adult-derived cardiac fibroblast (Lonza); and human fetal-derived cardiac fibroblast (Cell Applications, San Diego, CA). These cells were expansion cultured in a fibroblast culture medium (HFDM-1(+) medium (Cell Science & Technology Institute, Inc., Miyagi, Japan), and used for a transplantation experiment.

### <Cell sorting>

A fibroblast was primarily immunostained with human CD106 (VCAM-1)-biotin (Miltenyi Biotec), or human CD90-biotin (Miltenyi Biotec) and secondarily immunostained with anti-biotin microbeads (Miltenyi Biotec). CD106- and CD90-positive cells were collected from the stained cell by autoMACS (Miltenyi Biotec).

### <Production of chronic heart failure model rat>

All animal experimental protocols carried out in the present study were approved by the Ethical Review Committee of LSI Medience Corporation (Tokyo, Japan). All animals received refinement alternatives.

A nude rat (F344/N Jcl-rnu/rnu) (8-week-old) (male) was purchased from CREA Japan Inc. (Tokyo, Japan). After acclimation for one week or more, each animal was subjected to inhalation anesthesia with 2% isoflurane (anesthetic adjuvant; laughing gas: oxygen = 7:3) by use of an inhalation anesthesia apparatus for experimental animals (Soft Lander (SHIN-EI INDUSTRIES, INC., Saitama, Japan), and then the hair was clipped. Tracheal cannulation was rapidly carried out, and 0.5 to 2% isoflurane (anesthetic adjuvant; laughing gas: oxygen = 7:3) inhalation anesthesia gas was directly introduced into a respirator to maintain anesthesia. Under artificial respiratory management, the animal was fixed in a supine position, and thoracotomy was performed by longitudinally cutting, at costal cartilage, two or three ribs between the left third rib and the fifth rib. The operative field was expanded with a retractor, and then the pericardial membrane was detached to expose the heart. The left atrium was lifted up, and a thread was allowed to pass at a depth of about 2 mm for a length of 4 to 5 mm in the left ventricle by use of a weakly curved round needle with thread, for blood vessels (6-0: Nescosuture). Both ends of the thread were combined, a snare produced with a polyethylene tube was allowed to pass, and the thread was tightened with an artery clamp (snare method) to lead to coronary artery ischemia for 30 minutes. After 30 minutes, reperfusion was made and the condition was stabilized, and then the absence of bleeding was confirmed, thoracic cavity drainage was carried out and the muscle layer and the skin were sutured. The skin was subjected to subcuticular suture, and, when normal suture was carried out, suture removal was carried out with the postoperative condition being monitored. On the day before cell administration at one week after model production, echocardiographic measurement was carried out with an ultrasonic image diagnostic apparatus (Xario SSA-660A, Toshiba Medical Systems Corporation, Tochigi, Japan). An individual having a left ventricular ejection fraction (LVEF = (LVIDd3 - LVIDs3)/LVIDd3) of 55% or less was regarded as a chronic heart failure model, and subjected to a fibroblast administration experiment.

### <Administration of fibroblast to chronic heart failure model rat>

Various fibroblasts cryopreserved were thawed on the day of the administration test, diluted with high-glucose DMEM+10% NBCS, and a fibroblast suspension (2.0 × 10⁶ cells/50 µL in terms of the number of living cells) was administered to each individual. Anesthesia of each animal was maintained by the same procedure as in model production, and a total amount of 50 µL of the fibroblast suspension was administered with a catheter equipped with a 30-G needle at two divided positions in the infarct section under artificial respiratory management. After the condition was stabilized, the absence of leakage of the liquid administered or bleeding was confirmed, thoracic cavity drainage was carried out, and the muscle layer and the skin were sutured. The skin was subjected to subcuticular suture, and, when normal suture was carried out, suture removal was carried out with the postoperative condition being monitored.

### <Collection of heart and histological stain>

Each rat after 18 weeks from cell administration was sacrificed, and the heart was collected. The heart collected was fixed with 4% paraformaldehyde, thereafter a paraffin section was made by paraffin embedding and also a frozen section was made. The paraffin section was subjected to Sirius Red (SR) staining or hematoxylin-eosin (HE) staining, and used for *in-situ* hybridization. An adult-derived cardiac fibroblast administration group was subjected to imaging with a virtual slide scanner (NanoZoomer S210, Hamamatsu Photonics K.K., Shizuoka, Japan). A fetal-derived cardiac fibroblast administration group was subjected to imaging with an Aperio ScanScope slide scanner. An SR staining image was used to quantitatively determine a fibrotic region with Image J in the case of the adult-derived cardiac fibroblast administration group and with HALO (Indica Labs, Albuquerque, NM) in the case of the fetal-derived cardiac fibroblast administration group. An HE staining image was used to evaluate the following items by qualitative scoring: Inflammatory cell aggregation, proliferation of fibroblasts, fibrosis, lime deposition, and thinning of the left ventricular. The degree of pathological change was qualitatively evaluated with No change (Score 0), Mild (Score 1), Moderate (Score 2), and Severe (Score 3). In *in-situ* hybridization, staining was carried out with RNAscope Target Probe Rn-Lyve-1-C2 (Bio-Techne, Minneapolis, MN), according to the recommended protocol from the manufacturer. Here, probe treatment was performed with HybEZ Oven (Bio-Techne). The frozen section was subjected to immune tissue staining with an anti-cardiac troponin T(cTnT) antibody (Abcam, Cambridge, UK), an anti-prospero-related homeobox 1 (Prox-1) antibody (Proteintech, Rosemont, IL), and an anti-Vascular endothelial growth factor receptor 3 (VEGFR3) antibody (Bioss Antibodies, Woburn, MA), and imaged with an FV1200 confocal laser microscope (Olympus, Tokyo, Japan).

### <Statistical analysis>

Comparison of the average values between a NT group and a +CKs group was carried out by statistical analysis with Student's t-test.

<Example A1: increase in CD106-positive cell ratio of adult-derived cardiac fibroblast by contact of TNF-α and IL-4>

Human adult-derived cardiac fibroblasts were cultured in a cell culture dish, and a NT group and a +CKs group were provided. The human adult-derived cardiac fibroblasts in all the groups had a flat and spindle shape, and had a typical fibroblast-like shape (FIG. 1A).

The CD90, CD106, and CD90 and CD106 double-positive cell ratios (%) were evaluated by flow cytometry, thus those in the NT group were respectively 48.88% (CD90), 0.20% (CD106) and 0.14% (double-positive, DP), whereas those in the +CKs group were respectively 57.55% (CD90), 50.78% (CD106) and 34.76% (DP), and the CD90-positive cell ratio, the CD 106-positive cell ratio and the double-positive cell ratio (%) were considerably increased (FIG. 1B). It was shown from the present results that TNF-α and IL-4 were contacted with fibroblasts to result in increase in CD90, CD106, and double-positive cell ratios (%) of the fibroblasts.

### <Example A2: Increase in expression level(s) of ADM and/or HHEX in adult-derived cardiac fibroblast by contact with TNF-α and IL-4>

The whole RNA was extracted from each of the adult-derived cardiac fibroblasts in all the groups (NT group, +CKs group) and the quality of an RNA sample was checked, and thereafter the fluorescence intensity correlating with the number of mRNA copies was detected by the probe detection system (5'-nuclease method) and the relative value of the number of mRNA copies was calculated by a calibration curve method (N = 3). The gene expression levels of ADM and HHEX in the fibroblasts in all the groups were normalized with the gene expression level of β-Actin, and were represented by Fold increase under the assumption that the gene expression level(s) of ADM and/or HHEX in the NT group (average value) was 1.00. As a result, it was shown that the expression level of ADM was enhanced 2.30 times in the +CKs group and a significant difference in expression level of mRNA was confirmed (FIG. 2A). In this regard, the expression level of HHEX was shown to be enhanced 2.58 times in the +CKs group and a significant difference in expression level of mRNA was confirmed in all the groups (FIG. 2B). It was shown from these results that TNF-α and IL-4 were contacted with a fibroblast to enhance the gene expression level(s) of ADM and/or HHEX in the fibroblast.

### <Example A3: high cardiac lymphangiogenesis effect by adult-derived cardiac fibroblast contacted with TNF-α and IL-4>

A lymphatic endothelial cell-containing human heart vascular endothelial cell was expansion cultured in order to evaluate the lymphangiogenesis effect of an adult-derived cardiac fibroblast treated with TNF-α and IL-4. A double-positive lymphatic endothelial cell positive to VE-cadherin and PDPN as endothelial cell markers was used for a test with.

A human lymphatic endothelial cell collected from a heart-derived cell and any of various human adult-derived cardiac fibroblasts were co-cultured and the lymphatic vessel was observed by immunofluorescent staining, and thus it was revealed that, while an untreated adult-derived cardiac fibroblast (NT) exhibited discontinuous vessel formation with the lymphatic vessel divided, an adult-derived cardiac fibroblast (+CKs) cultured for 24 hours with the addition of TNF-α (50 ng/mL) and IL-4 (2 ng/mL) formed a continuous vessels not divided (FIG. 3A). Also from the results of quantitative analysis, +CKs provided significant elongation of the entire length of mature lymphatic vessel, and such elongation was 2.21 ± 0.76 times that in NT (FIG. 3B). A significant increase in number of junctions was also observed, and that in +CKs was increased 1.84 ± 0.65 times that in NT (FIG. 3B). It was revealed from the present results that an adult-derived cardiac fibroblast treated with TNF-α and IL-4 was a cell high in lymphangiogenesis effect.

### <Example A4: therapeutic effect of fibrosis exerted by adult-derived cardiac fibroblast contacted with TNF-α and IL-4 and made CD90 and CD106 double-positive>

Many prior studies have reported that lymphangiogenesis can treat scar formation (fibrosis) caused according to organ tissue failure. The following adult-derived cardiac fibroblasts were first prepared in order to confirm the effect (FIG. 4): untreated adult-derived cardiac fibroblast (A-HCF); adult-derived cardiac fibroblast cultured for 72 hours with the addition of TNF-α (50 ng/mL) and IL-4 (2 ng/mL) (uA-HCF); and a cell group collected by cell sorting a CD90 and CD106 double-positive cell population of uA-HCF, by MACS with CD90 as an index (uA90-HCF). For comparison with a naturally occurring CD90 and CD106 double-positive cardiac fibroblast, a fetal cardiac fibroblast was subjected to cell sorting by MACS with CD106 as an index and collected (F-VCF). The evaluation results of characteristics of various fibroblasts by FCM are illustrated in FIG. 4.

The following cells were administered to chronic heart failure model rats, and a fibrosis region was observed by Sirius Red staining (FIG. 5). Test 1: microscope observation of each group of A-HCF administration group; uA-HCF administration group; uA90-HCF administration group; Control group (group of administration of only the medium); and Sham group (group where only a sham surgery was performed and no heart failure occurred); was performed (FIGs. 5A and B). Test 2: microscope observation of each group of F-VCF administration group and Control group (group of administration of only the medium); was performed (FIGs. 5C and D). As a result, a fibroblast to which TNF-α (50 ng/mL) and IL-4 (2 ng/mL) were added provided a small area of a cardiac fibrosis region, and provided a remarkably small fibrotic region particularly in a group of administration of only a cell population made CD90 and CD106 double-positive by the effect of TNF-α and IL-4 (uA90-HCF). Interestingly, no therapeutic effect of a fibrotic region was confirmed in the results of administration in the group of naturally occurring CD90 and CD106 double-positive cardiac fibroblast (F-VCF) administration. From the foregoing results, the difference in functionality to fibrosis, between lymphangiogenesis fibroblasts artificially produced with the addition of TNF-α (50 ng/mL) and IL-4 (2 ng/mL), could be found. In addition, pathology assessment was performed based on HE staining images of groups where various adult-derived cardiac fibroblasts were administered, and then the results from a fibroblast with the addition of TNF-α (50 ng/mL) and IL-4 (2 ng/mL) tended to be reduced in scores of Proliferation of fibroblast, Fibrosis, and Lime deposition as in the SR staining results and the respective values of such scores were remarkably decreased particularly in the group of administration of only a cell population made CD90 and CD106 double-positive by the effect of TNF-α and IL-4 (uA90-HCF) (FIG. 6).

Next, a lymphatic vessel endothelial receptor 1 (Lyve-1) as a lymphocyte endothelial marker was stained in *in-situ* hybridization, to observe lymphatic endothelial cells recruited to the myocardial infarction nest. As a result, a fibroblast with the addition of TNF-α (50 ng/mL) and IL-4 (2 ng/mL) was high in lymphatic endothelial cell-recruiting effect (lymphangiogenesis effect), as in the results of SR staining and HE staining, and the effect was remarkably increased particularly in the group of administration of only a cell population made CD90 and CD106 double-positive by the effect of TNF-α and IL-4 (uA90-HCF) (FIG. 7).

### <Example A5: in vivo lymphangiogenesis effect by CD90 and CD106 double-positive cardiac fibroblast>

A chronic heart failure model rat was subjected to transepicardial intramyocardial administration of a fibroblast (A-HCF, uA-HCF, or uA90-HCF) or Vehicle Control (only a medium), and *in vivo* lymphangiogenesis in each group was observed (FIG. 8). As a result of immunohistochemical observation, localization of Prox-1 and VEGFR3 as lymphatic endothelial cell (LEC) markers could be confirmed only in the uA90-HCF administration group, but localization of LEC could not be confirmed in other groups (FIG. 8).

### <Example B: Example about fibroblast highly expressing ADM>

### <Culturing of human pulmonary fibroblast and production of fibroblast highly expressing ADM and increasing expression of VCAM-1>

A human adult pulmonary fibroblast was purchased from PromoCell (Heidelberg, Germany), and was expansion-cultured in a fibroblast culture medium (HFDM-1(+) medium (Cell Science & Technology Institute, Inc., Miyagi, Japan) to which 5% (v/v) NBCS (Newborn Calf Serum) was added. A human lung microvascular endothelial cell was purchased from Lonza (Basel, Switzerland) and expansion-cultured in an EBM-2 medium (Lonza). Bright field observation was performed with Leica DMi1 (Leica GmbH, Wetzlar, Germany).

In order to produce a fibroblast highly expressing ADM and increasing expression of VCAM-1, a human pulmonary fibroblast was expansion cultured in a cell culture dish, and a group of culturing without treatment (Control) and a group of treatment by two-agent mixing of TNF-α (50 ng/mL) (Peprotech, Cranbury, NJ) and IL-4 (2 ng/mL) (FUJIFILM Wako Pure Chemical Corporation, Osaka, Japan) (+Factor-X) were provided. All the groups were subjected to culturing for 1 day (24 hours) with HFDM-1(+) where 5% NBCS was added, as a basic medium.

### <Flow cytometry>

A human pulmonary fibroblast was subjected to immunofluorescent staining with human CD90-PE (Miltenyi Biotec, Bergisch Gladbach, Germany) and BV421 mouse anti-human CD106 (BD Biosciences, San Jose, CA). Isotype controls used were an REA control (S)-PE (Miltenyi Biotec) and a BV421 mouse IgG1 κ isotype control (BD Biosciences). In this regard, a mouse pulmonary fibroblast was subjected to immunofluorescent staining with mouse CD90.2-PE (Miltenyi Biotec) and mouse CD106 (VCAM-1)-APC (Miltenyi Biotec). Isotype controls here used were rat IgG2b-PE (Miltenyi Biotec) and rat IgG2a-APC (Miltenyi Biotec). The fibroblast was a living cell and subjected to reaction with an antibody at 4°C for 30 minutes. The fibroblast subjected to the immunofluorescent staining was analyzed by a flow cytometer (MACSQuant Analyzer 10, Miltenyi Biotec). After a cell region was identified by FSC-A and SSC-A, the positive cell ratio (%, in terms of the number of cells) to CD90 and CD106 proteins was evaluated.

### <Evaluation of gene expression level in pulmonary fibroblast>

Sequences (SEQ ID NOs: 17 to 18 (human VEGF-C primer), SEQ ID NOs: 4 to 5 (human ADM primer), SEQ ID NOs: 8 to 9 (human ACTB primer), SEQ ID NOs: 19 to 24 (mouse VEGF-C primer, mouse ADM primer, mouse GAPDH primer)) of a primer used were designed based on human mRNA sequences (VEGF-C, NM_005429.5 (SEQ ID NO: 13); ADM, NM_001124.3 (SEQ ID NO: 1); ACTB (β-Actin), NM_001101.5 (SEQ ID NO: 3)) and mouse mRNA sequence (VEGF-C,NM_009506.2 (SEQ ID NO: 14); ADM, NM_009627.2 (SEQ ID NO: 15); GAPDH,NM_001289726.1 (SEQ ID NO: 16)) published in NCBI so that the anneal temperature calculated by a nearest neighbor base-pair method was about 60°C and one or more introns were sandwiched. A 5'-nuclease probe sequence was also designed based on the published human and mouse mRNA sequence data, and the anneal temperature of the probe was designed to a temperature of 5°C or higher than that of the primer and the binding position of the probe was designed so as to be bound to a base between the binding position of the forward primer and the binding position of the reverse primer on the gene of a measurement subject. A dual quencher probe where a fluorescent dye 6-FAM was bound to the 5'-end of the probe, a quencher dye ZEN was bound at the 3'-side by nine bases from the end and a second quencher dye Iowa Black FQ was bound to the 3'-end was obtained, and a back ground signal in RT-PCR analysis was decreased (SEQ ID NO: 25 (human VEGF-C probe), SEQ ID NO: 10 (human ADM probe), SEQ ID NO: 12 (human ACTB probe), SEQ ID NOs: 26 to 28 (mouse VEGF-C probe, mouse ADM probe, mouse GAPDH probe)). Synthesis of all the above primers and probes was outsourced to Integrated DNA Technologies (Table 2).

**[Table 2]**

| Table 2. Sequence information of primer and probe | | |
|---|---|---|
| Gene | | Sequence |
| human VEGF-C | Forward Primer | 5'-gctacctcagcaagacgttatttg-3' (SEQ ID NO:17) |
| | Reverse Primer | 5'-atcggcaggaagtgtgattg-3' (SEQ ID NO:18) |
| | Probe | 56-FAM/aattacagt/ZEN/gcctctctctcaaggcccc/3IABkFQ (SEQ ID NO:25) |
| human ADM | Forward Primer | 5'-atgtacctgggttcgctcgc-3' (SEQ ID NO:4) |
| | Reverse Primer | 5'-ccacgactcagagcccactt-3' (SEQ ID NO:5) |
| | Probe | 56-FAM/ttcgaaact/ZEN/ccgacgcgacatcc/31ABkFQ (SEQ ID NO:10) |
| human β-Actin | Forward Primer | 5'-accttctacaatgagctgcg-3' (SEQ ID NO: 8) |
| | Reverse Primer | 5'-cctggatagcaacgtacatgg-3' (SEQ ID NO:9) |
| | Probe | 56-FAM/atctgggte/ZEN/atettetegeggttg/31ABkFQ (SEQ ID NO:12) |
| mouse VEGF-C | Forward Primer | 5'-gcttcttgtctctggcgtg-3' (SEQ ID NO:19) |
| | Reverse Primer | 5'-gacacagaccgcaactgc-3' (SEQ ID NO:20) |
| | Probe | 56-FAM/tctttgcct/ZEN/tcaaaagccttgacctcgcc/3IABkFQ (SEQ ID NO:26) |
| mouse ADM | Forward Primer | 5'-gggccagatactccttcgca-3' (SEQ ID NO:21) |
| | Reverse Primer | 5'-ctggcggtagcgtttgacac-3' (SEQ ID NO:22) |
| | Probe | 56-FAM/tccgaaaga/ZEN/agtggaataagtgggcgc/3IABkFQ (SEQ ID NO:27) |
| mouse GAPDH | Forward Primer | 5'-gaggccggtgctgagtatg-3' (SEQ ID NO:23) |
| | Reverse Primer | 5'-acccttttggctccaccct-3' (SEQ ID NO:24) |
| | Probe | 56-FAM/tcaagtggg/ZEN/ecccggcett/3IABkFQ (SEQ ID NO:28) |

The whole RNA was extracted from a pulmonary fibroblast by use of Qiagen RNeasy Plus Mini Kit (QIAGEN). Here, a fibroblast lysate was crushed with a QIA shredder (QIAGEN), and other operations including DNase treatment were made according to a recommended protocol from the kit manufacturer. The concentration of the whole RNA obtained was measured by measuring the absorbance at a wavelength of 260 nm with a Nanodrop One/One spectrophotometer (Thermo Fisher Scientific). The value of A260/A280, representing the degree of contamination of protein, was calculated at the same time, and A260/A280 > 2.0 was confirmed.

In real time quantitative RT-PCR, the reverse transcription reaction and RT-PCR were collectively performed with the same tube by use of One Step PrimeScript 3 RT-qPCR Mix, with UNG Kit (TaKaRa). The reaction was performed in a reaction liquid (total amount 15 µL) containing 1 × One Step PrimeScript 3 RT-qPCR Mix, with UNG, a 0.2 µM forward & reverse primer, a 0.2 µM specific probe, and 10 ng (human pulmonary fibroblasts) or 100 ng (mouse pulmonary fibroblasts) of a template RNA solution. The reverse transcription reaction was first performed by warming to 52°C for 5 minutes and thereafter heating was made at 95°C for 10 seconds for inactivation of a reverse transcription enzyme. Subsequently, a cycle for heating at 95°C for 5 seconds and at 60°C for 30 seconds was repeated 40 times, and the fluorescence intensity was measured with respect to each of such cycles. A set of reactions and measurements was performed with 7500 Rast Real-Time PCR (Thermo Fisher Scientific). An RNA solution extracted from a sample confirmed about expression of ADM and HHEX was concurrently used as a preparation for creation of a calibration curve, to perform the measurement by use of the RNA solution serially diluted at 4 stages from 1 µg by 10-fold. All the samples were subjected to the measurement at N = 3.

After completion of PCR, the baseline and the threshold of the fluorescence intensity in the resulting amplification curve were set with respect to each target gene, and the number of cycles (Ct value), at which the fluorescent signal reached the threshold, was determined with respect to each of the samples. Since the correlation coefficient and the amplification efficiency in the calibration curve of each gene were respectively 0.99 or more and 85% or more and the calibration curve was considered to be satisfactory, the Ct value of a subject sample was applied to the calibration curve and the mRNA content of the objective gene was calculated as a relative value. The relative value was normalized with the value of a housekeeping gene (β-Actin or GAPDH) calculated in the same way. The gene expression levels in fibroblasts were represented by Fold increase under the assumption that the gene expression level of Control, normalized with the housekeeping gene expression level, was 1.

### <Lymphatic vessel formation assay>

The lymphatic vessel formation assay was made by co-culturing a lymphatic endothelial cell collected from a human lung-derived cell and any of various human pulmonary fibroblasts, by a procedure known by those skilled in the art, and evaluating the lung lymphangiogenesis effect by each of various pulmonary fibroblasts (Non-Patent Document 15). Specifically, a lymphatic endothelial cell collected from a human lung-derived cell and any of various human adult-derived pulmonary fibroblasts were seeded at a concentration of 2.5 × 10⁵ cells/cm² and at a ratio of 1:9, and co-cultured in EBM-2 medium for 3 days. After the co-culturing, these cells were fixed with 4% paraformaldehyde, and subjected to immunofluorescent staining. Primary antibodies here used were an anti-VE-Cadherin rabbit polyclonal antibody (abcam, Cambridge, UK) and an anti-Vimentin mouse polyclonal antibody (abcam). Secondary antibodies here used were goat anti-rabbit IgG H&L (Alexa Fluor 488) (abcam) and goat anti-rabbit mouse IgG H&L (Alexa Fluor 647) (abcam). Nuclear staining was performed with a Hoechst 33258 solution (DOJINDO LABORATORIES., Kumamoto, Japan). A staining sample was imaged with IN Cell Analyzer 2200 (Cytiva, Marlborough, MA).

### <Isolation/primary culturing of mouse pulmonary fibroblast, and production of fibroblast highly expressing ADM and increasing expression of VCAM-1>

All animal experiments carried out in the present study were approved by the Ethical Review Committee of Innovation Center of NanoMedicine (Kanagawa, Japan) (animal experiment protocol approval number: A20-003). All animals received refinement alternatives.

Mouse C57BL/6J (male, 10-week-old) was purchased from Charles River Laboratories Japan, Inc. (Kanagawa, Japan). A mouse adult-derived pulmonary fibroblast was a primary cell collected from a lung tissue, and this primary cell was expansion cultured. Specifically, an adult mouse lung tissue (C57BL/6 mouse, 10-week-old) collected was minced by a scalpel into 1-mm square, 0.14 Wunsch units/mL of Liberase TM (Sigma Aldrich, St. Louis, MO) was added to perform enzyme treatment in an incubator at 37°C, thereby performing isolation/culturing of a primary pulmonary fibroblast. Here, the culture medium used was a DMEM/F12 medium to which 15% FBS was added. Bright field observation was performed with Leica DMi1 (Leica GmbH, Wetzlar, Germany).

For production of an adult-derived pulmonary fibroblast highly expressing ADM and increasing expression of VCAM-1 (VPF), a mouse adult-derived pulmonary fibroblast was expansion cultured in a cell culture dish, and a group of culturing without treatment (Control) and a group where recombinant mouse TNF-α (50 ng/mL) (Peprotech) and recombinant mouse IL-4 (2 ng/mL) (Peprotech) were treated by two-agent mixing (+Factor-X) were provided. All the groups were subjected to culturing for 3 days (72 hours) with DMEM/F12 medium to which 15% FBS was added, as a base medium, to thereby produce each adult-derived pulmonary fibroblast highly expressing ADM and increasing expression of VCAM-1 (VPF). The VPF was used in Examples B3 and B4 described below.

The lung was collected from a C57BL/6 mouse (female, 10-week-old) purchased from Charles River Laboratories Japan, Inc. (Kanagawa, Japan) and a pulmonary fibroblast (PF) was expansion cultured by primary culturing to further produce PF highly expressing ADM and increasing expression of VCAM1 (VPF), according to the same procedure. The VPF was used in Example B5 described below.

In the present description, drawings, and the like, the "mouse-derived PF" is also sometimes referred to as "mPF" and the "rat-derived PF" is also sometimes referred to as "rPF". The "mouse-derived VPF" is also sometimes referred to as "mVPF" and the "rat-derived VPF" is also sometimes referred to as "rVPF".

### <Production of mouse interstitial pneumonia/pulmonary fibrosis model>

A bleomycin-induced mouse C57BL/6J model was purchased from Charles River Laboratories Japan, Inc., in order to evaluate the therapeutic effect of an adult-derived pulmonary fibroblast highly expressing ADM and increasing expression of VCAM-1 (VPF) on interstitial pneumonia and pulmonary fibrosis. Induction of interstitial pneumonia and pulmonary fibrosis with bleomycin was carried out by a procedure known by those skilled in the art (Non-Patent Document 16). Specifically, a bleomycin solution (1 U/ml in 0.9% physiological saline solution) was packed in a liquid MicroSprayer (PennCentury, Wyndmoor, PA), and was transtracheally administered at a concentration of 2 mg/kg in 50 mL to the lung of a mouse (male, 9-week-old) under deep anesthesia, and the mouse after 7 days from bleomycin solution administration was regarded as an interstitial pneumonia/pulmonary fibrosis model.

### <Cell administration to mouse interstitial pneumonia/pulmonary fibrosis model and evaluation of therapeutic effect of VPF after administration>

A fibroblast was intravenously administered to an interstitial pneumonia/pulmonary fibrosis model mouse via a tail vein. Various fibroblasts were administered at 1.0 × 10⁵ cells/100 µL of physiological saline solution/body. mPF, mVPF or a physiological saline solution (Ctrl) as a control was administered to each mouse.

The therapeutic effect of each of the cells was examined by survival analysis by a Kaplan-Meier method and pathological evaluation. In Examples B3 and B4, each of the animals was sacrificed after 4 weeks of cell administration (after 28 days) corresponding to the end of the test and the lung was collected. The lung collected was fixed with 4% paraformaldehyde, and then subjected to paraffin embedding. A paraffin section was subjected to Sirius Red (SR) staining, and imaged with a virtual slide scanner (NanoZoomer S210, Hamamatsu Photonics K.K., Shizuoka, Japan). In Example B5, the animal was sacrificed after 2 weeks of cell administration (after 14 days) corresponding to the end of the test and the lung was collected. The lung collected was fixed with 4% paraformaldehyde, and then subjected to paraffin embedding. A paraffin section was subjected to Masson's Trichrome staining and then imaged with BZ-X710 (Keyence, Osaka, Japan). The image taken was subjected to quantitative determination of a fibrotic region by ImageJ software.

### <Primary culturing of rat pulmonary fibroblast and production of fibroblast highly expressing ADM and increasing expression of VCAM-1>

The lung was collected from a WKY/NCrlCrlj rat (female, 6-week-old) purchased from Charles River Laboratories Japan, Inc. (Kanagawa, Japan), and a rat pulmonary fibroblast (rPF) was expansion cultured by primary culture. Specifically, a rat lung tissue collected by the primary was minced by a scalpel into 1-mm square, and 0.14 Wunsch units/mL of Liberase TM (Sigma Aldrich, St. Louis, MO) was added to perform enzyme treatment in an incubator at 37°C, thereby performing expansion culturing in DMEM (Low Glucose).

rPF highly expressing ADM and increasing expression of VCAM-1 (rVPF) was produced by the same procedure as the above mVPF production method. Specifically, the production was made by adding rat recombinant TNF-α and IL-4 to rPF (final concentrations: 50 ng/mL TNF-α, 2 ng/mL IL-4), and performing culturing for 72 hours. The VCAM-1-positive cell ratio of rVPF was evaluated by flow cytometry with MACSQuant Analyzer (Miltenyi Biotec, Bergisch Gladbach, Germany).

### <Production of rat interstitial pneumonia/pulmonary fibrosis model>

Creation of a model rat having a pathological condition was outsourced to Charles River Laboratories Japan, Inc. (Kanagawa, Japan). A bleomycin solution (5 mg/kg physiological saline solution) was administered by single intratracheal atomization to a BN/CrlCrlj rat (male, 6-week-old) with liquid MicroSprayer, and an interstitial pneumonia/pulmonary fibrosis model rat was produced. This rat strain was selected according to the following reasons: (1) a BN rat and a WKY rat were inbred rats and were immunologically deviated, and selection of both the strains was widely used in an efficacy trial of an allogeneic cell medicine; and (2) interstitial pneumonia/pulmonary fibrosis model production in the present strain has been reported (Non-Patent Document 17).

### <Cell administration to rat interstitial pneumonia/pulmonary fibrosis model and evaluation of therapeutic effect after 2 weeks of administration>

The following types of cells were intravenously administered at 5.0 × 10⁵ cells/500 µL physiological saline solution/body to an interstitial pneumonia/pulmonary fibrosis model rat after 5 days from bleomycin solution administration. rPF, rVPF or a physiological saline solution (Ctrl) as a control was administered to each rat.

The therapeutic effect of each of the cells was examined by survival analysis by a Kaplan-Meier method and pathological evaluation. Each of the animals was sacrificed after 2 weeks of cell administration (after 14 days) corresponding to the end of the test and the lung was collected. The lung collected was fixed with 4% paraformaldehyde, and then subjected to paraffin embedding. A paraffin section was subjected to Masson's Trichrome staining and then imaged with BZ-X710 (Keyence, Osaka, Japan). The image taken was subjected to quantitative determination of a fibrotic region by ImageJ software.

### <Culturing of human pulmonary fibroblast and production of fibroblast increasing expression of VCAM-1>

A human adult pulmonary fibroblasts was purchased from PromoCell (Heidelberg, Germany), and expansion cultured in a fibroblast culture medium (HFDM-1(+) medium (Cell Science & Technology Institute, Inc., Miyagi, Japan) to which 5% (v/v) NBCS (Newborn Calf Serum) was added.

A human pulmonary fibroblast was expansion cultured in a cell culture dish in order to produce a fibroblast increasing expression of VCAM-1, and a group of culturing without treatment (hPF) and a group of treatment by two-agent mixing of TNF-α (50 ng/mL) (Peprotech, Cranbury, NJ) and IL-4 (2 ng/mL) (FUJIFILM Wako Pure Chemical Corporation, Osaka, Japan) (hPF+CKs) were provided. All the groups were subjected to culturing for 3 days (72 hours) with HFDM-1(+) where 5% NBCS was added, as a basic medium. hVPF was collected by immunostaining of hPF+CKs with anti-CD106 (VCAM-1)-Biotin, a human antibody (Miltenyi Biotec, Bergisch Gladbach, Germany) and Anti-Biotin microBeads (Miltenyi Biotec) and cell sorting by autoMACS pro Separator (Miltenyi Biotec).

### <Statistical analysis>

The experiment results were each expressed by average value ± standard deviation (SD). In a significant difference test, calculation was made by a logrank (Mantel-Cox) test and a logrank-trend test in survival analysis according to a Kaplan-Meier method, and by Student's t-test in other experiments. P < 0.05 was regarded as a significant difference.

Statistical analysis and cell characteristic analysis by flow cytometry, of a fibrotic region, were carried out by GraphPad Prism 9 for Windows 64-bit, Version 9.2.0 (332). In a syngeneic transplantation experiment to a mouse, ordinary one-way ANOVA was carried out and multiple comparisons based on Ctrl were carried out by a Dunnett's multiple comparisons test. In an allograft transplantation experiment to a rat, ordinary one-way ANOVA was carried out and multiple comparisons in groups were carried out by a Tukey`s multiple comparisons test. In analysis of human pulmonary fibroblast characteristics, 2-way ANOVA was carried out and multiple comparisons in groups were carried out by a Tukey`s multiple comparisons test. P < 0.05 was regarded as a significant difference in any test.

### <Example B 1: lymphangiogenesis ability of fibroblast highly expressing ADM and increasing expression of VCAM-1>

A human adult-derived pulmonary fibroblast was expansion cultured in a cell culture dish by adhesion culturing. It was shown from bright field observation that the human adult-derived pulmonary fibroblast cultured was a spindle-shaped fibroblast-like form (FIG. 9A). TNF-α (50 ng/mL) and IL-4 (2 ng/mL) were added to the human adult-derived pulmonary fibroblast cultured, by two-agent mixing (Factor-X), and cultured for 24 hours (FIG. 9B), and the positive cell ratios of CD90 and VCAM-1 (CD106) as fibroblast markers were evaluated by flow cytometry (FCM) (FIGs. 9C and 9D). While almost all human adult-derived pulmonary fibroblasts were CD90-positive and the CD90-positive cell ratio of an untreated human adult-derived pulmonary fibroblast (Control) was 96.00 ± 4.33%, and the CD90-positive cell ratio of a human adult-derived pulmonary fibroblast (+Factor-X) to which TNF-α and IL-4 were added was 95.56 ± 4.51%. While the CD106-positive cell ratio of Control was 6.03 ± 1.91%, the CD106-positive cell ratio of +Factor-X was 31.96 ± 5.78%. While CD90 and CD106 double-positive (Double (+)) cell ratio of Control was 5.86 ± 1.94%, that of +Factor-X was 31.25 ± 6.27%. It was revealed from these results that TNF-α and IL-4 significantly increased the CD106-positive cell ratio of a human pulmonary fibroblast.

Next, the gene expression levels of VEGF-C and ADM as representative lymphangiogenesis factors were evaluated by RT-qPCR in order to evaluate the lymphangiogenesis ability of the human adult-derived pulmonary fibroblast increased in CD106-positive cell ratio by Factor-X (FIG. 9E). As a result, the gene expression levels of VEGF-C and ADM were respectively highly expressed 2.99 ± 0.39 times and 2.46 ± 0.69 times by the addition of Factor-X, and a significant difference was found in both the groups. It was indicated from the foregoing results that a human pulmonary fibroblast enhanced in VCAM-1 expression level by TNF-α and IL-4 highly expressed a lymphangiogenesis gene and was a cell rich in lymphangiogenesis ability of the lung.

### <Example B2: lymphangiogenesis effect of fibroblast highly expressing ADM and increasing expression of VCAM-1>

A lymphatic endothelial cell-containing human lung microvascular endothelial cell, collected from a human lung-derived cell, was expansion cultured in order to evaluate the lymphangiogenesis effect of a human-derived adult-derived pulmonary fibroblast highly expressing ADM and increasing expression of VCAM-1 (VPF). From bright field observation, it was shown that the cell cultured exhibited a pavement-like form (FIG. 10A), and it was revealed by FCM analysis that the present cell was 99.71 ± 0.09% positive to VE-Cadherin as an endothelial cell marker (N = 3) and was 96.77 ± 0.37% positive to PDPN as a lymphatic endothelial cell marker (N = 3) (FIG. 10B). A double-positive lymphatic endothelial cell positive to VE-Cadherin as an endothelial cell marker and PDPN as a lymphatic endothelial cell marker was used for testing.

A lymphatic endothelial cell collected from a human lung-derived cell, and any of various human adult-derived pulmonary fibroblasts were co-cultured and the lymphatic vessel was observed by immunofluorescent staining, and it was thus revealed that, while an untreated adult-derived pulmonary fibroblast exhibited discontinuous vessel formation with the lymphatic vessel divided, an adult-derived pulmonary fibroblast (+Factor-X) cultured for 24 hours with the addition of TNF-α (50 ng/mL) and IL-4 (2 ng/mL) formed continuous vessels not divided (FIG. 10C). It was revealed from the present results that a pulmonary fibroblast treated with TNF-α and IL-4 was a cell high in lymphangiogenesis effect.

### <Example B3: therapeutic effect of interstitial pneumonia/pulmonary fibrosis by fibroblast highly expressing ADM and increasing expression of VCAM-1>

A syngeneic transplantation experiment was designed in order to evaluate the therapeutic effect of interstitial pneumonia/pulmonary fibrosis by an adult-derived pulmonary fibroblast high in lymphangiogenesis effect, treated with TNF-α and IL-4. A lung-derived fibroblast was collected from a C57BL/6 adult mouse, and expansion cultured. It was shown from bright field observation that the primary adult-derived pulmonary fibroblast cultured was a spindle-shaped fibroblast-like form (FIG. 11A). TNF-α (50 ng/mL) and IL-4 (2 ng/mL) were added to a mouse adult-derived pulmonary fibroblast cultured, and cultured for 3 days, and the CD106-positive cell ratio of the mouse adult-derived pulmonary fibroblast was evaluated by FCM (FIG. 11B). As a result, while the ratio in Control was 84.89 ± 11.42% (N = 3), that in the fibroblast to which Factor-X was added was 96.52 ± 3.32% (N = 3). It was found from the present results that a mouse pulmonary fibroblast was relatively high in CD106-positive cell ratio even in a normal condition, as compared with a human pulmonary fibroblast, and TNF-α and IL-4 could increase the CD106-positive cell ratio regardless of species.

The gene expression levels of VEGF-C and ADM in an adult-derived pulmonary fibroblast were evaluated by RT-qPCR in order to evaluate lymphangiogenesis ability of a mouse adult-derived pulmonary fibroblast highly expressing ADM and increasing expression of VCAM-1 (VPF), and thus the respective gene expression levels of VEGF-C and ADM were 4.11 ± 0.59 times (N = 3) high and 1.70 ± 0.27 times (N = 3) high by the addition of TNF-α (50 ng/mL) and IL-4 (2 ng/mL) (Factor-X), as in those in a human adult-derived pulmonary fibroblast, and a significant difference was found in both the groups (FIG. 11C). It was revealed from the present results that a mouse pulmonary fibroblast significantly highly expressed a lymphangiogenesis gene by TNF-α and IL-4, as in a human pulmonary fibroblast, and that mouse-derived VPF was also a cell rich in lymphangiogenesis ability.

### <Example B4: therapeutic effect of interstitial pneumonia/pulmonary fibrosis by fibroblast highly expressing ADM and increasing expression of VCAM-1>

Cell administration was applied to a mouse interstitial pneumonia/pulmonary fibrosis model according to an experimental schedule described in FIG. 12A in order to evaluate the therapeutic effect of an adult-derived pulmonary fibroblast highly expressing ADM and increasing expression of VCAM-1 (VPF) on interstitial pneumonia/pulmonary fibrosis. The following three model animal groups were provided: mouse adult-derived pulmonary fibroblast administration group (+PFs, N=8), mouse VPF administration group (+VPFs, N=10), and physiological saline administration group (Control, N=10).

The effect of each of the groups on vital prognosis was evaluated with survival analysis by a Kaplan-Meier method, and thus the survival rates on Day 28 were respectively 40.00% (Control), 37.50% (+PFs), and 80.00% (+VPFs) (FIG. 12B). The median survival times were respectively 14.5 days (Control), 14 days (+PFs), and undefinable due to survival of 80% of individuals (+VPFs), and an improvement in vital prognosis by VPF administration was confirmed.

The lung was collected after 4 weeks of cell administration, a lung tissue from each of the groups was subjected to Sirius Red staining, and the alveoli structure and the degree of progress of fibrosis were evaluated by pathological observation. While no clear partition of the alveoli structure was observed and a fibrotic region was confirmed mainly around the bronchi in the Control group and the +PFs group, a clear alveoli partition was observed and no fibrotic region was observed in the +VPFs group (FIG. 12C).

### <Example B5: mVPF administration to mouse interstitial pneumonia/pulmonary fibrosis model and evaluation of therapeutic effect after 2 weeks of administration>

Furthermore, mPF, mVPF or a physiological saline solution (Ctrl) was administered to each mouse interstitial pneumonia/pulmonary fibrosis model mouse and the survival rate after 2 weeks from the administration was compared and evaluated, and thus two death cases were confirmed on each of Day 10 and Day 14 and a survival rate of 63.64% was exhibited in the Ctrl group (7/11 cases) (FIG. 13A). In this regard, one death case was confirmed on each of Day 7 and Day 9 and the survival rate was 71.43% in the mPF administration group (5/7 cases). Furthermore, no death case was confirmed at all and the survival rate was 100% in the mVPF administration group (5/5 cases). It was indicated from the present results that a high survival rate improvement effect of mVPF on interstitial pneumonia/pulmonary fibrosis was exerted.

Next, Masson's Trichrome staining of a lung tissue collected after 14 days from cell administration was carried out and pathological observation was carried out, and it was thus revealed that the smallest fibrosis region was exhibited and a normal alveoli structure was exhibited in the mVPF administration group (FIG. 13B). A fibrotic region in each of the groups was quantitatively evaluated, and it was thus revealed that the smallest fibrotic region was exhibited in the mVPF administration group and the region was significantly small as compared with that in the Ctrl group (FIG. 13C). While the fibrotic region was significantly increased in the Ctrl group as compared with that of an individual on 0 days from cell administration, no significant difference was found in other groups (mPF administration group and mVPF administration group). It was also found that the fibrotic region was small in the mVPF administration group as compared with that in the mPF administration group. It was found from these results that syngeneic transplantation/administration of mVPF served to increase the survival rate of a mouse interstitial pneumonia/pulmonary fibrosis model by bleomycin administration and suppress the progress of a pathological condition of fibrosis.

### <Example B6: isolation of rat pulmonary fibroblast and production of VCAM-1-positive rat pulmonary fibroblast>

A rat pulmonary fibroblast (rPF) was isolated from a WKY/NCrlCrlj rat, and expansion cultured in a DMEM (Low Glucose) medium (FIG. 14A). Next, rat recombinant TNF-α (final concentration: 50 ng/mL) and IL-4 (final concentration: 2 ng/mL) were added to rPF and production of rVPF was carried out (FIG. 14B). FCM analysis was made, and the VCAM-1 (CD106)-positive cell ratio of rPF was 25.18%, and on the other hand, rVPF produced with the addition of TNF-α and IL-4 exhibited a positive cell ratio of 80.02% (FIG. 14C). Accordingly, it was found that rPF exhibited responsiveness to TNF-α and IL-4 as in mPF and rVPF could be produced.

### <Example B7: administration of rVPF to rat interstitial pneumonia/pulmonary fibrosis model and evaluation of therapeutic effect after 2 weeks of administration>

rPF, rVPF or a physiological saline solution (Ctrl) as a control was administered to each rat interstitial pneumonia/pulmonary fibrosis model mouse and the survival rate after 2 weeks from the administration was compared and evaluated, and thus no death and moribund individual was confirmed after 2 weeks from cell or physiological saline solution administration in any group in a rat model, unlike a mouse model, and thus no difference in survival rate among the groups was observed.

On the other hand, Masson's Trichrome staining of a lung tissue collected after 14 days from cell administration was carried out and pathological observation was carried out, and thus a large difference among the groups was confirmed. It was found that a broad fibrosis confirmed in an image of the lung (BLM) before cell administration at 5 days after bleomycin administration was further exacerbated by physiological saline solution administration (Ctrl) and caused fibrosis and abnormal structure formation found in the entire lung (FIGs. 15A and 15B). In this regard, fibrosis and abnormal structure formation in the lung were only locally confirmed and a fibrotic region was significantly small than that in Ctrl, in the rPF and rVPF administration groups. In particular, it was found that the rVPF administration group exhibited a significantly decreased fibrotic region as compared with such a region before cell administration and had the decreasing effect of such a fibrotic region (FIGs. 15A and 15B). Those were similar to the results of the syngeneic transplantation experiment to a mouse, and it was indicated that VPF also served as an autologous cell medicine and an allogeneic cell medicine to exert an amelioration/prevention effect of pulmonary fibrosis.

### <Example B8: cell characteristic analysis of human pulmonary fibroblast increasing expression of VCAM-1>

A human adult-derived pulmonary fibroblast (hPF) was expansion cultured in a cell culture dish by adhesion culturing. hPF+CKs was produced by adding TNF-α (50 ng/mL) and IL-4 (2 ng/mL) by two-agent mixing (Factor-X), to hPF cultured, and performing culturing for 72 hours. hPF+CKs was subjected to cell sorting with VCAM-1 (CD106) as an index, and hVPF was produced.

The positive cell ratios of CD90 and VCAM-1 (CD106) as fibroblast markers were evaluated by flow cytometry (FCM) (FIG. 16A). While almost all human adult-derived pulmonary fibroblasts were CD90-positive and the CD90-positive cell ratio of hPF was 99.68 ± 0.06% (N = 5), the CD90-positive cell ratio of hPF+CKs was 98.81 ± 0.51% (N = 5). The CD90-positive cell ratio of hVPF was 99.08 ± 0.41% (N = 5), and no significant difference was found in each group (FIG. 16B).

On the other hand, while the VCAM-1 (CD106)-positive cell ratio of hPF was 15.49 ± 2.52% (N = 5), the CD106-positive cell ratio of hPF+CKs was 31.38 ± 3.41% (N = 5). The CD106-positive cell ratio of hVPF was 98.11 ± 1.01% (N = 5), and it was found that hPF+CKs was significantly high in CD106-positive cell ratio as compared with hPF and hVPF was further significantly high in positive cell ratio as compared with hPF+CKs (FIG. 16B).

The CD90 and CD106 double-positive cell ratio (DP) was similar, and, while the DP of hPF was 15.38 ± 2.59% (N = 5), the DP of hPF+CKs was 31.21 ± 3.30% (N = 5). The DP of hVPF was 97.47 ± 1.18% (N = 5), and it was found that hPF+CKs was significantly higher in DP than hPF and hVPF was further significantly higher in DP than hPF+CKs (FIG. 16B).

It was revealed from the foregoing that a fibroblast treated with TNF-α and IL-4 highly expressed ADM and increased the VCAM-1-positive cell ratio, in a fibroblast, and a fibroblast highly expressing ADM and enhancing expression of VCAM-1 had lymphangiogenesis ability enhanced and thus promoted continuous lymphatic vessel formation. It was then revealed that the fibroblast, in particular, syngeneic cell of the fibroblast derived from the lung was intravenously administered to thereby significantly improve the vital prognosis of mouse interstitial pneumonia/pulmonary fibrosis and the present cell was effective for amelioration of fibrosis. Furthermore, it was indicated that an adult-derived fibroblast highly expressing ADM and increasing expression of VCAM-1, in particular, VPF also served as an autologous cell medicine and an allogeneic cell medicine to exert the amelioration/prevention effect of pulmonary fibrosis. Therefore, intravenous administration of an adult-derived fibroblast highly expressing ADM and increasing expression of VCAM-1, in particular, VPF can be expected in a novel therapeutic product for pulmonary fibrosis including IPF known as a poor-prognosis disease.

## Claims

1. A pharmaceutical composition comprising a fibroblast highly expressing ADM and/or HHEX.

2. The pharmaceutical composition according to claim 1, wherein the fibroblast is an adult-derived fibroblast.

3. The pharmaceutical composition according to claim 1 or 2, wherein gene expression level(s) of ADM and/or HHEX in the fibroblast are/is 1.20 times or higher as compared with a control fibroblast.

4. The pharmaceutical composition according to any one of claims 1 to 3, for treating fibrosis.

5. The pharmaceutical composition according to any one of claims 1 to 4, for amelioration of a condition of lymphangiogenesis ability reduced.

6. The pharmaceutical composition according to any one of claims 1 to 5, for amelioration of a disorder of homeostasis in a tissue fluid.

7. The pharmaceutical composition according to any one of claims 1 to 6, for reduction of edema.

8. The pharmaceutical composition according to any one of claims 1 to 7, for amelioration of a disorder of transportability of lipid and/or vitamin.

9. The pharmaceutical composition according to any one of claims 1 to 8, for activation of an immunological surveillance mechanism.

10. The pharmaceutical composition according to any one of claims 1 to 9, wherein the pharmaceutical composition is an injectable composition.

11. The pharmaceutical composition according to any one of claims 1 to 9, wherein the fibroblast is constructed as a planar or three-dimensional cellular tissue.

12. The pharmaceutical composition according to any one of claims 1 to 11, wherein the fibroblast is a cardiac fibroblast.

13. A method for producing a fibroblast highly expressing ADM, comprising:
contacting TNF-α and IL-4 with a fibroblast; and
culturing the fibroblast contacted, under a condition where ADM is capable of being highly expressed.

14. The method according to claim 13, wherein the fibroblast highly expresses VEGF-C.

15. The method according to claim 13 or 14, wherein the fibroblast is CD106-positive.

16. The method according to claim 15, comprising selecting or concentrating a fibroblast highly expressing ADM, by use of an anti-CD106 antibody.

17. The method according to any one of claims 13 to 16, wherein the fibroblast is a pulmonary fibroblast.

18. A fibroblast highly expressing ADM, obtained by the method according to any one of claims 13 to 17.

19. A cell population of fibroblast comprising the fibroblast according to claim 18.

20. A fibroblast highly expressing ADM.

21. The fibroblast according to claim 20, wherein the fibroblast highly expresses VEGF-C.

22. The fibroblast according to claim 20 or 21, wherein the fibroblast is CD106-positive.

23. The fibroblast according to any one of claims 20 to 22, wherein gene expression level(s) of VEGF-C and/or ADM in the fibroblast are/is 1.20 times or higher as compared with a control fibroblast.

24. The fibroblast according to any one of claims 20 to 23, wherein the fibroblast is a pulmonary fibroblast.

25. A cell population of fibroblast comprising the fibroblast according to any one of claims 20 to 24.

26. A cell population comprising a CD106-positive pulmonary fibroblast, wherein the proportion (based on the number of cells) of the CD106-positive pulmonary fibroblast in the total fibroblast contained in the cell population is more than 18.01%.

27. A pharmaceutical composition comprising the fibroblast according to any one of claims 18 and 20 to 24 or the cell population according to any one of claims 19 and 25 to 26.

28. The pharmaceutical composition according to claim 27, for treating a pulmonary disease.

29. The pharmaceutical composition according to claim 28, wherein the pulmonary disease is pulmonary fibrosis or interstitial pneumonia.

30. The pharmaceutical composition according to claim 27, for amelioration of a condition of lymphangiogenesis ability reduced.

31. The pharmaceutical composition according to any one of claims 27 to 30, wherein the pharmaceutical composition is an injectable composition.

32. The pharmaceutical composition according to any one of claims 27 to 30, wherein the fibroblast or the cell population is constructed as a planar or three-dimensional cellular tissue.
